# EUROPEAN PATENT SPECIFICATION

(11) **EP 2 322 600 B1**
(45) Date of publication and mention of the grant of the patent: **21.03.2012**
(21) Application number: 10011579.9
(22) Date of filing: 30.08.2005
(51) Int. Cl.: C12N 5/071, A61L 27/38

(54) **Cultured three dimensional tissues and uses thereof**
Dreidimensionale kultivierte Gewebe und deren Verwendung
Tissus tridimensionnels de culture et utilisations de ceux-ci

(30) Priority: 30.08.2004 US 606072 P; 17.06.2005 US 691731 P
(43) Date of publication of application: 18.05.2011
(62) Divisional of application: 05794944.8
(73) Proprietor: Theregen, Inc., San Francisco CA 94105 (US)
(72) Inventor: Naughton, Gail K., San Diego California 92101 (US)
(74) Representative: Horner, Martin Grenville

(56) References cited:
- EP-A2- 0 358 506
- US-A1- 2001 014 475
- US-A1- 2003 007 954

## Description

### 1. BACKGROUND

Use of a three dimensional scaffold for culturing cells, such as fibroblasts, allows the cultured cells to sustain long term proliferation and elaborate various growth factors. Three dimensional cell cultures of this type are described in US Patent Nos. 5,266,480 and 5,443,950 and are available commercially as Dermagraft®. Because of their unique properties, these three dimensional tissues have found applications as skin replacements and as culture systems for organ specific cells, such as bone marrow and liver cells (see, *e.g*., US Patent Nos. 5,460,939, 5,541,107 and 5,559,022). US2003/007954 discloses and evidences the suitability of a cultured 3-D tissue comprising (i) cells, (ii) connective tissue proteins secreted by the cells and (iii) a non-living scaffold, for promoting angiogenesis of heart tissue by attaching said 3-D tissue to the heart.

Typically, the three dimensional tissues are prepared on a mesh and applied as a patch onto the tissue or organ. For applications to internal tissues, such as cardiac tissues, a surgical procedure is used to access the site, and the patch attached to the tissue with glues, staples, sutures, or other means. However, any surgical procedure is invasive and can lead to medical complications, such as infection and bleeding. Thus, it is desirable to provide alternative approaches to such treatments.

### 2. SUMMARY

The present disclosure provides compositions and medicaments according to claim 1 or 2 administrable by minimally invasive methods, for use in treating ischemic heart. The compositions comprise microparticles dimensioned or so dimensioned as to permit injection of the composition or medicament into tissues, where the microparticles comprise in-vitro-cultured three dimensional tissue comprising a scaffold (synonymously, "framework" or "support") formed of a biocompatible, non-living material. The compositions may be administered by injection or use of a catheter.

In some embodiments, the three dimensional scaffold comprises nonwoven filaments matted to provide a three dimensional scaffold. The nonwoven filaments comprise biodegradable filaments, or blends of biodegradable and non-biodegradable filaments, that when cultured in the presence of cells form particulates having dimensions suitable for injection.

In other embodiments, the three dimensional scaffold comprises a woven or braided material having dimensions suitable for administration by injection, delivery by a catheter, or use as a suture. In some embodiments, the woven or braided three dimensional scaffold comprise a cord or braided sheath having interstices for the attachment and proliferation of cells. In some embodiments, the interstices form a luminal space, such as in a tube, formed by a braided sheath.

The three dimensional scaffolds are cultured with stromal cells comprising fibroblasts, and optionally other cells of tissue specific origin. Cells cultured with the microparticles form three dimensional networks of cells, where the cells attach to and extend out from the microparticle scaffold. Genetically engineered cells may also be used to form the three dimensional tissues.

In some aspects, the compositions are used as a source of or to deliver various growth factors produced by the three dimensional tissues, including VEGF and Wnt proteins. Specific Wnt proteins elaborated by the culture include, among others, Wnt5a, Wnt7a, and Wnt 11. In other embodiments, the compositions comprise the conditioned media obtained from the three dimensional tissues, where the conditioned media comprises the repertoire of growth factors produced by the cultured cells.

The compositions and medicaments of this invention may be used to treat ischemic heart. In some embodiments, the compositions are used to promote repair and regeneration of ischemic cardiac tissue. The compositions and medicaments may be used to promote repair and healing at anastomosis sites, such as that arising from surgery.

### 3. BRIEF DESCRIPTION OF THE FIGURES

FIG 1 shows MTT, DNA and VEGF assay results of smooth muscle cells grown on Alkenmes® microparticles for 18 days.

FIG. 2 shows cultured beads with the contour of cells surrounding the beads. The spheres become more translucent as the beads inside degrade.

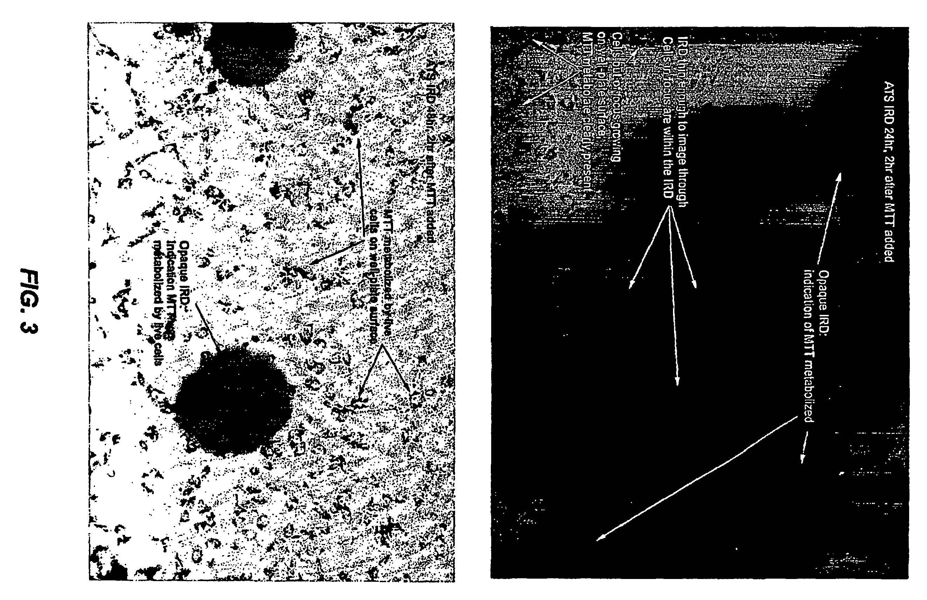

FIG. 3 shows viability of cells after 24 and 48 hrs of storage under shipping conditions, indicating that cell remained viable after such treatment.

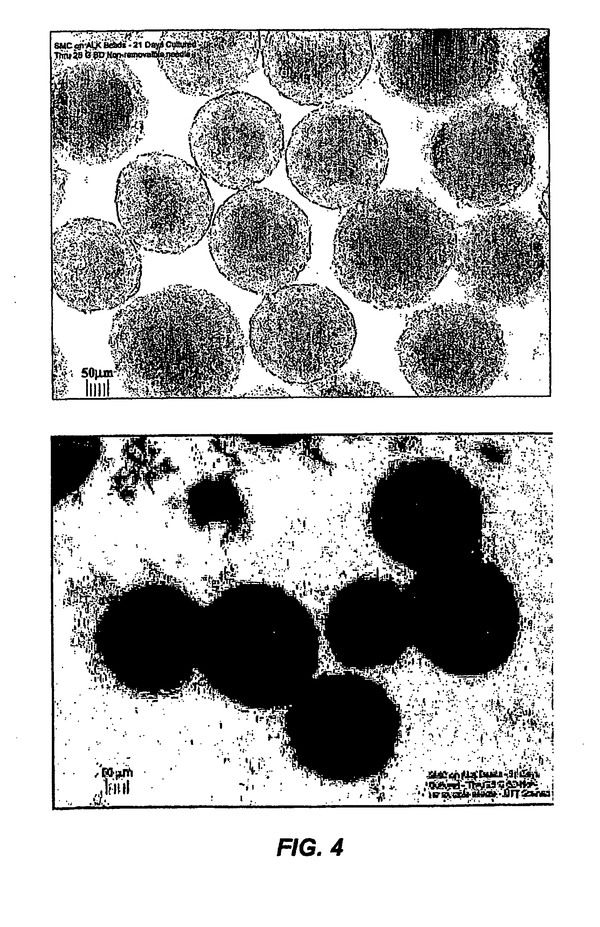

FIG. 4 shows that all cells cultured with microparticles remained viable after 24 hrs of incubation following passage through a 24 gauge needle and that the cultured microparticles remained their original, spherical shape following treatment.

FIG. 5 shows unseeded Prodesco braided suture types: (1) 24 carriers, 12 axials with a high braid angle (250 ppi); (2) 24 carriers, 12 axials with a low braid angle (200 ppi); (3) 8 carriers, 12 axials; and (4) 8 carriers, 24 axials.

FIG. 6 shows MTT stained sutures described in FIG. 5 after 2 weeks of culture with canine smooth muscle cells (SMC).

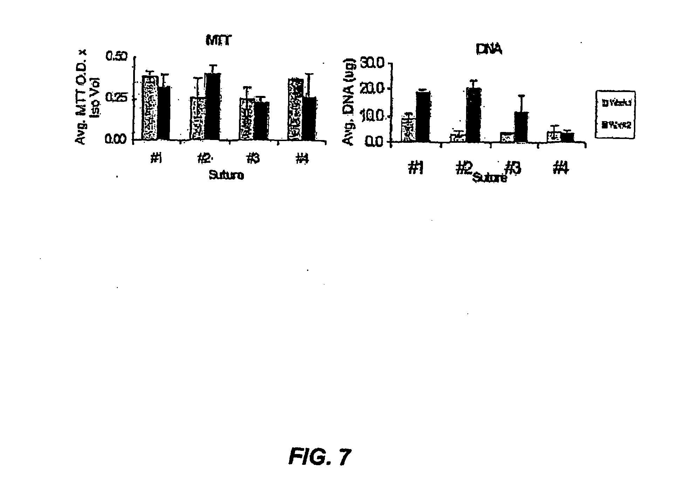

FIG. 7 shows MTT and DNA staining of Prodesco sutures after one and two weeks of culture with canine SMC.

FIG. 8 shows MTT staining of braided threads before and after exposure to shipping conditions, indicating that the cells remain viable in the suture material.

FIG. 9 shows presence and viability of cells on braided threads passed through both cardiac and peripheral muscle.

FIGS. 10A and 10B depict the injection of cultured beads from a 24 gauge needle equipped Hamilton Syringe into ischemic hindlimb tissue.

FIGS 11A and 11B are photographs of ischemia only treated animals two weeks after inducing ischemia.

FIG. 12A and 12B are photographs of two week explants of three dimensional tissues formed on microparticles, showing evidence of limited new microvessel formation (black arrows) in ischemic limbs treated with SMC grown on Alkermes® beads.

FIGS. 13A and 13B show new microvessel formation (black arrows) surrounding braided threads after 14 days of implantation.

### 4. DETAILED DESCRIPTION

The present disclosure provides injectable cell culture or tissue compositions and/or medicaments according to claims 1 and 2 that produce a suite (synonymously, a "repertoire", a "fingerprint", a "signature", a "cocktail") of growth factors capable of promoting repair and regeneration of damaged tissue. These three dimensional tissues, also referred to as "engineered minimally invasive tissue" or "engineered minimally invasive construct" are small yet robust enough to be delivered by minimally invasive methods, such as by injection or by a catheter. Thus, the cultured three dimensional tissues are dimensioned for or so dimensioned as to permit penetration into tissues. The scaffolds or framework are composed of a biocompatible, non-living material, which may be fashioned in various forms to produce a tissue penetrating composition. The scaffold or framework may be of any material and/or shape that: (a) allows cells to attach to it (or can be modified to allow cells to attach to it); and (b) allows cells to grow in more than one layer (*i.e*., form a three dimensional tissue). Accordingly, the scaffolds can be a support or framework on which cells can invade, divide, and occupy interstitial spaces to form a three dimensional tissue type structure. In some embodiments, the scaffolds are by themselves not attached to each other but can function as substrates for cell attachment such that the cells along with the scaffold material act together to fonn a three dimensional tissue structure. The tissue engineered constructs are typically characterized by the presence of cells that are extended or stretched on the three dimensional scaffold and an extracellular matrix environment similar to larger three dimensional tissue constructs (see, *e.g*., US Patent No. 5,830,708). Additionally, the cells of the three dimensional tissues produce a repertoire or cocktail of growth factors that can affect the proliferation and differentiation of surrounding cells. These compositions find various uses in treating heart ischemia, inducing vascularization (*e.g*., angiogenesis), and promoting tissue regeneration.

For the descriptions of the compositions of three dimensional tissues, the following meanings will apply.

"Degradable" refers to erodability or degradability of a compound or composition under conditions of use. Biodegradable also refers to absorbability or degradation of a compound or composition when administered *in vivo* or under *in vitro* conditions. Biodegradation may occur through the action of biological agents, either directly or indirectly.

"Biocompatible" refers to compounds or compositions and their corresponding degradation products that are relatively non-toxic and are not clinically contraindicated for administration into a tissue or organ.

"Growth factor" refers to any soluble, extracellular matrix-associated, or cell-associated factor that promotes cell proliferation, cell differentiation, tissue regeneration, cell attraction, wound repair, and/or any developmental cell proliferative process. A biological activity of a growth factor refers to one or all activities associated with a particular growth factor.

"Tissue damage" refers to abnormal conditions in a tissue or organ resulting from an insult to a tissue. Types of insult include, but are not limited to, disease, surgery, injury, aging, chemicals, heat, cold, and radiation.

### 5.1 Three Dimensional Tissues Formed With Microparticles

The framework (synonymously "scaffold") for the cell cultures comprises particles that, in combination with the cells, form a three dimensional tissue. The cells attach to the particles and to each other to form a three dimensional tissue. The complex of the particles and cells is of sufficient size to be administered into tissues or organs, such as by injection or catheter. As used herein, a "microparticle" refers to a particle having size of nanometers to micrometers, where the particles may be any shape or geometry, being irregular, non-spherical, spherical, or ellipsoid. Microparticles encompass microcapsules, which are microparticles with one or more coating layers. In some embodiments, the microparticles comprise microspheres. As used herein "microspheres" refer to microparticles with a spherical geometry. A microsphere, however, need not be absolutely spherical, as deviations are permissible for generating the three dimensional tissues.

The size of the microparticles suitable for the purposes herein can be determined by the person skilled in the art. In some embodiments, the size of microparticles suitable for the three dimensional tissues may be those administrable by injection. In some embodiments, the microparticles have a particle size range of at least about 1 µm, at least about 10 µm, at least about 25 µm, at least about 50 µm, at least about 100 µm, at least about 200 µm, at least about 300 µm, at least about 400 µm, at least about 500 µm, at least about 600 µm, at least about 700 µm, at least about 800 µm, at least about 900 µm, at least about 1000 µm. The characteristics and size of the microparticles can be readily determined using a variety of techniques, such as scanning electron microscopy, light scattering, or differential scanning calorimetry.

In some embodiments in which the microparticles are made of biodegradable materials, the particles are made to have a defined half-life under a defined biological condition. "Mean half life" as used in the context of microparticles refers to the mean time required for the particles to degrade to half the initial mass of a microparticle. The half-life of the microparticles may vary depending on various parameters, including, among others, type of biodegradable polymers or combination of polymer, the polymer porosity (*e.g*., porous or nonporous), molecular weight of the polymers, microparticle geometry, and level of polymer crosslinking. Choosing microparticles with a short or long half-life may be varied by the practitioner depending on the frequency of administration, the longevity of the cells following administration, and the time that the three dimensional tissue is effective in producing the desired effect, such as elaboration of a suite of growth factors. Thus in some embodiments, the microparticles in the three dimensional tissues have a mean half-life of about 14 days, a mean half-life of about 28 days, a mean half-life of about 90 days, or a mean half-life of about 180 days. As will be apparent to the skilled artisan, the half life may be made shorter or longer to achieve the desired therapeutic properties of the compositions.

In some embodiments, to vary its half-life, microparticles comprising two or more layers of different biodegradable polymers may be used. In some embodiments, at least an outer first layer has biodegradable properties for forming the three dimensional tissues in culture, while at least a biodegradable inner second layer, with properties different from the first layer, is made to erode when administered into a tissue or organ.

In some embodiments, the microparticles are porous microparticles. Porous microparticles refers to microparticles having interstices through which molecules may diffuse in or out from the microparticle. In other embodiments, the microparticles are non-porous microparticles. A nonporous microparticle refers to a microparticle in which molecules of a select size do not diffuse in or out of the microparticle.

Microparticles for use in the compositions are biocompatible and have low or no toxicity to cells. Suitable microparticles may be chosen depending on the tissue to be treated, type of damage to be treated, the length of treatment desired, longevity of the cell culture *in vivo*, and time required to form the three dimensional tissues. The microparticles may comprise various polymers, natural or synthetic, charged (*i.e*., anionic or cationic) or uncharged, biodegradable, or nonbiodegradable. The polymers may be homopolymers, random copolymers, block copolymers, graft copolymers, and branched polymers.

In some embodiments, the microparticles comprise non-biodegradable scaffolds. Non-biodegradable microcapsules and microparticles include, but not limited to, those made of polysulfones, poly (acrylonitrile-co-vinyl chloride), ethylene-vinyl acetate, hydroxyethylmethacrylate-methyl-methacrylate copolymers. These are useful to provide tissue bulking properties or in embodiments where the microparticles are eliminated by the body.

In some embodiments, the microparticles comprise degradable scaffolds. These include microparticles made from naturally occurring polymers, non-limiting example of which include, among others, fibrin, casein, serum albumin, collagen, gelatin, lecithin, chitosan, alginate or poly-amino acids such as poly-lysine. In other embodiments, the degradable microparticles are made of synthetic polymers, non-limiting examples of which include, among others, polylactide (PLA), polyglycolide (PGA), poly (lactide-co-glycolide) (PLGA), poly(caprolactone), polydioxanone trimethylene carbonate, polyhybroxyalkonates (*e.g*., poly (hydroxybutyrate)), poly(ethyl glutamate), poly(DTH iminocarbony(bisphenol A iminocarbonate), poly(ortho ester), and polycyanoacrylates.

In some embodiments, the microparticles comprise hydrogels, which are typically hydrophilic polymer networks filled with water. Hydrogels have the advantage of selective trigger of polymer swelling. Depending on the composition of the polymer network, swelling of the microparticle may be triggered by a variety of stimuli, including pH, ionic strength, thermal, electrical, ultrasound, and enzyme activities. Non-limiting examples of polymers useful in hydrogel compositions include, among others, those formed from polymers of poly (lactide- co-glycolide), poly (N-isopropylacrylamide); poly (methacrylic acid-g-polyethylene glycol); polyacrylic acid and poly (oxypropylene-co-oxyethylene) glycol; and natural compounds such as chrondroitan sulfate, chitosan, gelatin, fibrinogen, or mixtures of synthetic and natural polymers, for example chitosan-poly (ethylene oxide). The polymers may be crosslinked reversibly or irreversibly to form gels adaptable for forming three dimensional tissues (see, *e.g*., U. S. Patent No. 6,451,346; 6,410,645; 6,432,440; 6,395,299; 6,361,797; 6,333,194; 6,297,337; Johnson et al., 1996, Nature Med. 2:795.

In some embodiments, another type of particles useful in the compositions and methods of this disclosure comprise nanoparticles, which are generally microparticles of about 1 um or less in diameter or size. In some embodiments, the nanoparticles have a particle size range of at least about 10 nm, at least about 25 nm, at least about 50 nm, at least about 100 nm, at least about 200 nm, at least about 300 nm, at least about 400 nm, at least about 500 nm, at least about 600 nm, at least about 700 nm, at least about 800 nm, at least about 900 nm, at least about 1000 nm. Nanoparticles are generally made from amphiphilic diblock, triblock, or multiblock copolymers as is known in the art. Polymers useful in forming nanoparticles include, but are limited to, polylactide (PLA; see Zambaux et al., 1999, J. Control Release 60: 179-188), polyglycolide, poly(lactide-co-glycolide), blends of poly(lactide-co-glycolide) and polycarprolactone, diblock polymer poly(1-leucine-block-1-glutamate), diblock and triblock poly(lactic acid) (PLA) and poly(ethylene oxide) (PEO) (De Jaeghere et al., 2000, Pharm. Dev. Technol. 5:473-83), acrylates, arylamides, polystyrene. As described for microparticles, nanoparticles may be non-biodegradable or biodegradable. Nanoparticles may be also be made from poly (alkylcyanoacrylate), for example poly (butylcyanoacrylate), in which proteins are absorbed onto the nanoparticles and coated with surfactants (*e.g*., polysorbate 80).

In some embodiments, specifically excluded are microparticles made of hydrogels and other swellable polymers. In other embodiments, specifically excluded are microparticles made of hyaluronic acid.

Various methods for making microparticles are well known in the art, including solvent removal process (see, *e.g*., U. S. Patent No. 4,389,330); emulsification and evaporation (Maysinger et al., 1996, Exp. Neuro. 141: 47-56; Jcffrey et al., 1993, Pharm. Res. 10: 362-68), spray drying, and extrusion methods. Methods for making nanoparticles are similar to those for making microparticles and include, among others, emulsion polymerization in continuous aqueous phase, emulsification-evaporation, solvent displacement, and emulsification-diffusion techniques (see Kreuter, 1991, J., "Nano-particle Preparation and Applications," in Microcapsules and nanoparticles in medicine and pharmacy, pg. 125-148, (M. Donbrow, ed.) CRC Press, Boca Rotan, FL.

### 5.2 Three Dimensional Tissues Formed With Matted Fibers

In some embodiments, the scaffold or framework of the three dimensional tissue is made from a nonwoven network of biodegradable, biocompatible filaments that form particulate structures when incubated with cells in a culture medium. Generally, the nonwoven filaments comprise matted natural or synthetic polymeric or fibrous material formed into a three dimensional network, such as in the form of a web, felt, or pulp. The nonwoven framework provides a three dimensional structure that allows cells to proliferate and form cell-cell contacts to generate a tissue-like structure and elaborate the suite of growth factors having the desired biological properties. The fibers act as struts, defining the boundaries of the interstitial spaces; cells attach to the fibers and proliferate to fill the void spaces in the nonwoven network. While not being bound by any theory of action, the particulate composition of the matted fibers and cells appears to form as the fibers or polymers degrade under culture conditions and pockets or isolated masses of nonwoven filaments and cells detach from original network of fibers or polymers.

The nonwoven network may be formed in some embodiments by compressing intertwined or entangled fibers or polymers. In some embodiments, the filament junctions or crosspoint may be bonded to provide mechanical strength and/or a three dimensional lattice. Although the scaffold or framework are nonwoven, it is to be understood that two or more plies of nonwoven fabric may be attached together by stitching, or a binder, such an adhesive to form the three dimensional framework. The layers or plies are typically positioned in a juxtaposed or a surface-to-surface relationship. Different density of matted fibers may be used to alter the properties of the three dimensional framework, for instance, to add mechanical strength or increase the time required for degradation of the scaffold (see, *e.g*., U.S. Patent No. 6,077,526).

The fibers may be of uniform length or random length and may be made from natural or synthetic fibers, or combinations thereof. The filaments may also comprise a uniform diameter or may be comprised of filaments of differing diameters. In embodiments in which the nonwoven filaments comprise blends of compatible fibers, the mixtures may be fibers of differing mechanical strength, degradation rate, and/or adhesiveness. Filaments of shorter length may produce the particulate compositions with shorter culturing times but which dissipates fasters when administered while filaments of longer length may produce particulate compositions with longer culturing times but which dissipates more slowly upon administration (see, *e.g.,* Wang et al., 1997, J. Biomater. Sci. Polymer Edn. 9(1):75-87. The choice of filaments to form the non-woven framework is readily determined by the person skilled in the art.

The nonwoven network of filaments may be made of various fibers or polymers, natural or synthetic. Biodegradable filaments for making the nonwoven three dimensional framework may employ fibers and polymers used to make other types of scaffold structures described herein. The polymers may be homopolymers, random copolymers, block copolymers, graft copolymers, and branched polymers. Non-limiting examples of biodegradable natural polymers include among others, catgut, elastin, fibrin, hyaluronic acid, cellulose derivatives, and collagen. Non-limiting examples of biodegradable synthetic polymers include, among others, polylactide, polyglycolide, poly(e-caprolactone), poly(trimethylene carbonate) (TMC), and poly(p-dioxanone), and copolymers, such as poly(lactide-co-glycolide), poly(e-caprolactone-co-glycolide), poly(glycolide-co-trimethylene carbonate), poly(alkylene diglycolate), polyoxaesters, and copolymers made of PGA/PLA/TMC or any combination thereof in any percent combination. Descriptions for the preparation of such polymers and fibers are provided in various reference works and publications, such as Sorensen et al., 1968, Preparative Methods of Polymer Chemistry, Wiley, NY; Biodegradable Polymers As Active Agent Delivery Systems, (Chasin et al., eds.) Marcel Decker Inc., NY, 1997; and US Patent Nos. 6,866,860; 6,703,477; 5,348,700; 5,066,772; 4,481,353; 4,243,775; 4,429,080; and 4,157,357).

In some embodiments, the nonwoven three dimensional framework may comprise a combination of polymers (*i.e*., polymer blends) so long as they do not interfere with formation of the three dimensional tissues or the biodegradable characteristics of the compositions. Blends of the polymers may provide flexibility in providing the desired characteristics of particulate formation in culture, mechanical strength, durability when administered *in vivo*, and tissue bulking properties.

In some embodiments, the nonwoven three dimensional framework may further comprise non-biodegradable polymers, as further described below. Non-biodegradable polymers may be used to provide mechanical strength to and durability to the nonwoven network of biodegradable polymers. In some embodiments, the non-degradable polymers have lengths suitable for passage through an injection needle and/or allow formation of particulates of three dimensional tissues.

The nonwoven scaffold may be made by conventional techniques known in the art. Filaments, such a fibers or polymers of various lengths are made and then formed into a web or entangled matt, and the filaments optionally bonded within the web or matt by an adhesive or by mechanical frictional forces. For forming the particulate compositions, the nonwoven filaments are inoculated with the cells, as described below, and cultured in presence of the cells until portions of the filaments detach and form isolated or detached particles of scaffold, and cells. In some embodiments, formation of injectable particulates may be accelerated by mechanical action. This may be carried out in various ways, such as by passing the compositions through an orifice (*i.e*., needle) or gentle mechanical shearing. Preparation of the compositions will be well within the capabilities of the skilled artisan.

### 5.3 Three Dimensional Tissues Formed As Threads or Sutures

In some embodiments, the three dimensional scaffold is formed from multiple filaments, polymers or fibers that are braided, twisted, or woven, or otherwise arranged into a cord or a thread like structure that can be administered or inserted into tissues or organs. The scaffold comprises interstitial spaces that allow cells to attach and proliferate to form a three dimensional culture of living cells. In some embodiments, the braided or woven thread is suitable for use as a surgical suture material.

The cord or suture may be made in a range of conventional forms or constructions to have the interstitial spaces for invasion and attachment of cells and their proliferation. Generally, the openings and/or interstitial spaces of the cord scaffold should be of an appropriate size to allow the cells to stretch across the openings or spaces. Without intending to be bound by theory, maintaining actively growing cells stretched across the scaffold appears to enhance production of the suite of growth factors that appear to facilitate the desired activities described herein. If the openings are too small, the cells may rapidly achieve confluence but be unable to easily exit from the mesh. These trapped cells may exhibit contact inhibition and cease production of the appropriate factors desirable to support proliferation and maintain long term cultures. If the openings are too large, the cells may be unable to stretch across the opening, which may decrease stromal cell production of the appropriate factors desired to support proliferation and maintain long term cultures. Typically, the interstitial spaces are at least about 140 um, at least about 150 µm, at least about 180 um, at least about 200 µm, or at least about 220 um. However, depending upon the three-dimensional structure and intricacy of the scaffold, other sizes can work equally well. In fact, any shape or structure that allows the cells to stretch, replicate, and grow for a suitable length of time to elaborate the growth factors described herein can be used.

In some embodiments, the filaments are woven to form a luminal space for the proliferation of cells. The internal luminal space, which is a void space prior its occupation by cells, may or may not be occupied by a core filament. Although the luminal space may comprise varying geometric structures, luminal spaces in the braided structures may be in the form of a tube that runs lengthwise along the cord or sheath. Where a core is present, the sheath forms a jacket around the core. Different types of braids are known in the art. A spiral braid having different braiding angles may be made into cords with different tensile strengths. The core, when present, can be of various constructions, including, among others, a single filament or multiple filaments (see, *e.g*., US Patent No. 6,045,571), twisted or plied, and comprise a material that is the same or different from the sheath.

The cord or suture may be made from various materials described above for preparing other three dimensional scaffolds and frameworks. Homopolymers, random copolymers, block copolymers, and branched polymers may be used to form the cord or sutures. Non-limiting examples of biodegradable materials include, among others, polylactide (PLA), polyglycolide (PGA), poly(lactide-co-glycolide) (PLGA), polyethylene terephtalate (PET), polycaprolactone, dioxanone, poly(trimethylene carbonate) (TMC), poly(alkylene oxalate), polyoxaesters, copolymers made of PGA/PLA/TMC or any combination thereof in any percent combination, catgut suture material, collagen (*e.g*., equine collagen foam), hyaluronic acid, and compatible mixtures or blends thereof (see, *e.g*., US Patent No. 6,632,802; Biomedical Polymers, (Shalaby et al., eds.) Verlag, 1994; US Patent No. 6,177,094; US Patent No. 5,951,997).

In other embodiments in which additional structural integrity, durability, and/or tensile strength is desired, filaments of nonbiodegradable materials may be used. Non-limiting examples of nonbiodegradable materials include silk, polyesters (*e.g*., polyester terephthalate, dacron), polyamides (*e.g*., nylons), polyethylene, polypropylene, cellulose, polystyrene, polyacrylates, polyvinyls, polytetrafluoroethylenes (PTFE), expanded PTFE (ePTFE), and polyvinylidine fluoride. Other polymers will be apparent to the skilled artisan.

In other embodiments, the three dimensional scaffold or framework is a combination of different biodegradable filaments or combinations of biodegradable and non-biodegradable materials. A non-biodegradable material provides stability to the structures during culturing and increase the tensile strength when used as a suture material. The biodegradable material may be coated onto the non-biodegradable material or woven, braided or formed into a mesh. For instance, a sheath may be made of biodegradable filaments while the core is made of nonbiodegradable filaments. Various combinations of biodegradable and non-biodegradable materials may be used. An exemplary combination is poly(ethylene therephtalate) (PET) fabrics coated with a thin biodegradable polymer film (poly(lactide-co-glycolide)).

The three dimensional framework may be braided into a cord, such as a suture, by techniques conventional in the art. Processes and methods for producing braided or knitted tubular sheaths, including various types of sutures, are described in, *e.g*., in U.S. Pat. Nos. 3,773,919; 3,792,010; 3,797,499; 3,839,297; 3,867,190; 3,878,284; 3,982,543; 4,047,533; 4,060,089; 4,137,921; 4,157,437; 4,234,775; 4,237,920; 4,300,565; 4,523,591; 5,019,093, 5,059,213; 5,133,738; 5,181,923; 5,261,886; 5,306,289; 5,314,446; 5,456,697; 5,662,682; 6,045,071; 6164339; and 6,184,499. An exemplary method for forming filaments, such as PLGA, is a melt spinning process. Biocompatible bioabsorbable multifilament sutures are also available commercially under such tradenames as Dexon®, Vicryl®, and Polysorb® from various suppliers, such as Ethicon, Inc. (Somerville, NJ, USA), United States Surgical (Norwalk, CT, USA), and Prodesco (Perkasie, PA, USA)

Cords and braided sutures may be subjected to further processing, such as hot stretching, scouring, annealing, coating, tipping, cutting, needle attachment, packaging and sterilization prior to inoculation with the cells as necessary or desirable. To alter the mechanical characteristics, the filament can be stretched to reorient the molecule chains in the polymer. Annealing can be carried out to fix the characteristics of the filament, such as to maintain the polymer orientation, alter tensile strength, and fix geometric stability of the filaments.

The cord or braid may be of various axial diameters or dimensions depending on the desired application. Braided or woven frameworks may have smaller diameters when used as sutures for holding tissues together while larger diameters may be used when administered into tissues or organs for repair of tissue damage. In various embodiments, the diameters of the braided or woven frameworks range from about 0.05mm, 0.10mm, 0.2mm, 0.5mm, 1mm, 1.5mm, or about 2mm. It is to be understood that the diameters may be smaller or larger depending on the clinical application, the desired tensile strength, and the amount of cells attached to the framework.

### 5.4 Cells and Culture Conditions

For forming the three dimensional tissues, the biocompatible materials forming the scaffolds are inoculated with the appropriate cells and grown under suitable conditions to generate a three dimensional tissues. In various embodiments, the scaffold or framework material may be pre-treated prior to inoculation with cells to enhance cell attachment to the framework. For example, prior to inoculation with stromal cells, nylon screens are treated in some embodiments with 0.1 M acetic acid, and incubated in polylysine, fetal bovine serum, and/or collagen to coat the nylon. In some embodiments, polystyrene is analogously treated using sulfuric acid. In other embodiments, the growth of cells may be further enhanced by adding to the framework, or by coating the framework with proteins (*e.g*., collagens, elastin fibers, reticular fibers) glycoproteins, glycosaminoglycans (*e.g*., heparan sulfate, chondroitin-4-sulfate, chondroitin-6-sulfate, dermatan sulfate, keratan sulfate, etc.), a cellular matrix, and/or other materials, such as glycopolymer, such as (poly[N-p-vinylbenzyl-D-lactoamide], PVLA).

For use in medical applications, the three dimensional framework may be sterilized prior to inoculation with the cells. Sterilization methods include physical as well as chemical methods or a combination of such methods. A useful physical method of inactivating infectious agents is radiation (*e.g*., γ-radiation, UV light, electron-beam irradiation, etc.) or steam sterilization (for heat stable, non-degradable polymers). In other embodiments, a chemical process is used. An exemplary chemical for this purpose in ethylene oxide.

For forming the three dimensional tissues, the biocompatible materials forming the scaffolds are inoculated with the appropriate cells and grown under suitable conditions to promote formation of a three dimensional tissues. Cells can be obtained directly from a donor, from cell cultures made from a donor, or from established cell culture lines. In some embodiments, cells can be obtained in quantity from any appropriate cadaver organ or fetal sources. In some embodiments, cells of the same species, and optionally the same or similar immunohistocompatibility profile, may be obtained by biopsy, either from the subject or a close relative, which are then grown to confluence in culture using standard conditions and used as needed. The characterization of the donor cells with respect to the immunohistocompatibility profile are made in reference to the subject being administered the compositions.

Accordingly, in some embodiments, the cells are autologous. Because the three dimensional tissues derive from recipient's own cells, the possibility of an immunological reaction against the administered cells and/or products produced by the cells may be minimized. In some embodiments, the cells may be initially cultured on two-dimensional surfaces typically used in cell culture (*e.g*., plates) prior to seeding the three dimensional framework.

In other embodiments, the cells are obtained from a donor who is not the intended recipient of the compositions. The relation of the donor to the recipient is defined by similarity or identity of the multihistocompatibility complex (MHC). In some embodiments, the donor cells are syngeneic cells in that the cells derive from a subject who is genetically identical at the MHC to the intended recipient. In other embodiments, the cells are allogeneic cells in that the cells derive from a subject who is of the same species as the intended recipient but whose MHC complex is different. Where the cells are allogeneic, the cells may be from a single donor or comprise a mixture of cells from different donors who themselves are allogeneic to each other. In further embodiments, the cells are xenogenic cells in that the cells are derived from a species different than the intended recipient.

The cells inoculated onto the framework are stromal cells comprising fibroblasts, with or without other cells, as further described below. In some embodiments, the cells are stromal cells that are typically derived from connective tissue, including, but not limited to: (1) bone; (2) loose connective tissue, including collagen and elastin; (3) the fibrous connective tissue that forms ligaments and tendons, (4) cartilage; (5) the extracellular matrix of blood; (6) adipose tissue, which comprises adipocytes; and, (7) fibroblasts.

Stromal cells can be derived from various tissues or organs, such as skin, heart, blood vessels, skeletal muscle, liver, pancreas, brain, foreskin, which can be obtained by biopsy (where appropriate) or upon autopsy.

The fibroblasts can be from a fetal, neonatal, adult origin, or a combination thereof. In some embodiments, the stromal cells comprise fetal fibroblasts, which can support the growth of a variety of different cells and/or tissues. As used herein a fetal fibroblast refers to fibroblasts derived from fetal sources. As used herein neonatal fibroblast refers to fibroblasts derived from newborn sources. Under appropriate conditions, fibroblasts can give rise to other cells, such as bone cells, fat cells, and smooth muscle cells and other cells of mesodermal origin. In some embodiments, the fibroblasts comprise dermal fibroblasts. As used herein, dermal fibroblasts refers to fibroblasts derived from skin. Normal human dermal fibroblasts can be isolated from neonatal foreskin. These cells are typically cryopreserved at the end of the primary culture.

A three-dimensional tissue can be made using stem and/or progenitor cells, either alone, or in combination with any of the cell types discussed herein. Exemplary stem and progenitor cells include, by way of example and not limitation, non-human embryonic stem cells, hematopoietic stem cells, neuronal stem cells, epidermal stem cells, and mesenchymal stem cells. In some embodiments, excluded from the cell cultures are mesenchymal stem cells.

In some embodiments, a "specific" three-dimensional tissue can be prepared by inoculating the three-dimensional scaffold with cells derived from a particular organ, *i.e*., skin, heart, and/or from a particular individual who is later to receive the cells and/or tissues grown in culture in accordance with the methods described herein.

As discussed above, additional cells may be present in the culture with the stromal cells. These additional cells may have a number of beneficial effects, including, among others, supporting long term growth in culture, enhancing synthesis of growth factors, and promoting attachment of cells to the three dimensional scaffold. Additional cell types include as non-limiting examples, smooth muscle cells, cardiac muscle cells, endothelial cells, skeletal muscle cells, endothelial cells, pericytes, macrophages, monocytes, nerve cells, islet cells, and adipocytes. Such cells may be inoculated onto the three-dimensional framework along with fibroblasts. These additional cells may be derived from appropriate tissues or organs, including, by way of example and not limitation, skin, heart, blood vessels, skeletal muscle, liver, pancreas, and brain. In other embodiments, one or more other cell types, excluding fibroblasts, are inoculated onto the three-dimensional scaffold. In still other embodiments, the three-dimensional scaffolds are inoculated only with fibroblast cells.

Cells useful in the methods and compositions described herein can be readily isolated by disaggregating an appropriate organ or tissue. For example, the tissue or organ can be disaggregated mechanically and/or treated with digestive enzymes and/or chelating agents that weaken the connections between neighboring cells and thereby disperse the tissue into a suspension of individual cells without appreciable cell breakage. Enzymatic dissociation can be accomplished by mincing the tissue and treating the minced tissue with any of a number of digestive enzymes either alone or in combination. Non-limiting examples of enzymes include, among others, trypsin, chymotrypsin, collagenase, clastase, and/or hyaluronidasc, DNase, and pronasc. Mechanical disruption can also be accomplished by a number of methods including, but not limited to, the use of grinders, blenders, sieves, homogenizers, pressure cells, or insonators. For a review of tissue disaggregation techniques, see Freshney, Culture of Animal Cells. A Manual of Basic Technique, 2nd Ed., A.R. Liss, Inc., New York, 1987, Ch. 9, pp. 107-126.

Once the tissue has been reduced to a suspension of individual cells, the suspension can be fractionated into subpopulations from which the fibroblasts and/or other stromal cells and/or other cell types can be obtained. Standard techniques for cell separation and isolation include, by way of example and not limitation, cloning and selection of specific cell types, selective destruction of unwanted cells (negative selection), separation based upon differential cell agglutinability in the mixed population, freeze-thaw procedures, differential adherence properties of the cells in the mixed population, filtration, conventional and zonal centrifugation, centrifugal elutriation (counter-streaming centrifugation), unit gravity separation, countercurrent distribution, electrophoresis and fluorescence-activated cell sorting. For a review of clonal selection and cell separation techniques, see Freshney, *supra,* Ch. 11 and 12, pp. 137-168.

After inoculation of the three dimensional scaffolds, the cell culture is incubated in an appropriate nutrient medium and incubation conditions that supports growth of cells into the three dimensional tissues. Many commercially available media such as Dulbecco's Modified Eagles Medium (DMEM), RPMI 1640, Fisher's, Iscove's, and McCoy's, may be suitable for supporting the growth of the cell cultures. The medium may be supplemented with additional salts, carbon sources, amino acids, serum and serum components, vitamins, minerals, reducing agents, buffering agents, lipids, nucleosides, antibiotics, attachment factors, and growth factors. Formulations for different types of culture media are described in various reference works available to the skilled artisan (*e.g.,* Methods for Preparation of Media, Supplements and Substrates for Serum Free Animal Cell Cultures, Alan R. Liss, New York (1984); Tissue Culture: Laboratory Procedures, John Wiley & Sons, Chichester, England (1996); Culture of Animal Cells, A Manual of Basic Techniques, 4th Ed., Wiley-Liss (2000). Incubation conditions will be under appropriate conditions of pH, temperature, and gas (*e.g*., O₂, CO₂, etc) that support growth of cells. In some embodiments, the three-dimensional cell culture can be suspended in the medium during the incubation period in order to maximize proliferative activity and generate factors that facilitate the desired biological activities of the conditioned media. In addition, the culture may be "fed" periodically to remove the spent media, depopulate released cells, and add new nutrient source. During the incubation period, the cultured cells grow linearly along and envelop the filaments of the three-dimensional scaffold before beginning to grow into the openings of the scaffold.

The three dimensional tissues described herein have extracellular matrix that is present on the scaffold or framework. In some embodiments, the extracellular matrix comprises various collagen types, different proportions of which can affect the growth of the cells that come in contact with the three dimensional tissues. The proportions of extracellular matrix (ECM) proteins deposited can be manipulated or enhanced by selecting fibroblasts which elaborate the appropriate collagen type. This can be accomplished in some embodiments using monoclonal antibodies of an appropriate isotype or subclass that are capable of activating complement and which define particular collagen types. In other embodiments, solid substrates, such as magnetic bead, may be used to select or eliminate cells that have bound antibody. Combination of these antibodies can be used to select (positively or negatively) the fibroblasts which express the desired collagen type. Alternatively, the stroma used to inoculate the framework can be a mixture of cells which synthesize the appropriate collagen types desired. The distribution and origins of the exemplary type of collagen are shown in Table I.

**Table I: Distributions and Origins of Various Types of Collagen**

| **Collagen Type** | **Principle Tissue Distribution** | **Cells of Origin** |
|---|---|---|
| I | Loose and dense ordinary connective tissue; collagen fibers | Fibroblasts and reticular cells; smooth muscle cells |
| | Fibrocartilage | |
| | Bone | Osteoblast |
| | Dentin | Odontoblasts |
| II | Hyaline and elastic cartilage | Chondrocytes |
| | Vitreous body of the eye | Retinal cells |
| III | Loose connective tissue; reticular fibers | Fibroblasts and reticular cells |
| | Papillary layer of dermis | |
| | Blood vessels | Smooth muscle cells; endothelial cells |
| IV | Basement membranes | Epithelial and endothelial cells |
| | Lens capsule of the eye | Lens fiber |
| V | Fetal membranes; placenta | Fibroblasts |
| | Basement membranes | |
| | Bone | |
| | Smooth muscle | Smooth muscle cells |
| VI | Connective tissue | Fibroblasts |
| VII | Epithelial basement membranes; anchoring fibrils | Fibroblasts; keratinocytes |
| VIII | Cornea | Corneal fibroblasts |
| IX | Cartilage | |
| X | Hypertrophic cartilage | |
| XI | Cartilage | |
| XII | Papillary dermis | Fibroblasts |
| XIV (undulin) | Reticular dermis | Fibroblasts |
| XVII | P 170 bullous pemphigoid antigen | Keratinocytes |

During culturing of the three-dimensional tissues, proliferating cells may be released from the framework and stick to the walls of the culture vessel where they may continue to proliferate and form a confluent monolayer. To minimize this occurrence, which may affect the growth of cells, released cells may be removed during feeding or by transferring the three-dimensional cell culture to a new culture vessel. Removal of the confluent monolayer or transfer of the cultured tissue to fresh media in a new vessel maintains or restores proliferative activity of the three-dimensional cultures. In some embodiments, removal or transfers may be done in a culture vessel which has a monolayer of cultured cells exceeding 25% confluency. Alternatively, the culture in some embodiments is agitated to prevent the released cells from sticking; in others, fresh media is infused continuously through the system. In some embodiments, two or more cell types can be cultured together either at the same time or one first followed by the second (*e.g*., fibroblasts and smooth muscle cells or endothelial cells).

In some embodiments, the cells are cultured with the scaffold material in cell culture bags. An exemplary culturing device of this type is available commercially under the tradename Vuelife bags (American Fluoroseal Corp., Gaithersburg, MD, USA) and described in US Patent No. 4,847,462; and US Patent No. 4,945,203. Use of cell culture bags simplifies culturing and cryopreservation, as well as shipping.

In some embodiments, the three dimensional tissue may be prepared in bioreactors, such as those described in US Patent No. 5,763,267; 5,827,729; 6,008,049; 6,060,306; 6,121,042; and 6,218,182 Impellers in the bioreactors may be modified to limit attachment of the three dimensional tissues to the hubs. In addition, the working volume of impellers may be reduced by shortening the impeller shafts, thereby providing flexibility in culturing the three dimensional tissues.

In some embodiments, the three dimensional tissues are devoid of viable cells. Use of three dimensional tissues lacking viable cells also appears to promote repair and regeneration of tissues (see, *e.g*., U.S. Application Ser. No. 10/214,750, filed Aug 7, 2002). Three dimensional tissues prepared on microparticles, nowoven filaments, or braided scaffolds may be treated in various ways, such as cytotoxic agents, freezing, radiation, etc., to eliminate or kill viable cells to produce these compositions.

In various embodiments, the three dimensional tissues may be defined by a characteristic set, fingerprint, repertoire, or suite of cellular products produced by the cells, such as growth factors. In the three dimensional tissues specifically exemplified herein, the cell cultures are characterized by expression and/or secretion of the factors given in Table II

**Table II: Three Dimensional Tissue Expressed Factors**

| **Growth Factor** | **Expressed by Q-RT-PCR** | **Secreted Amount Determined by ELISA** |
|---|---|---|
| VEGF | 8 x 10⁶ copies/ug RNA | 700 pg/10⁶ cells/day |
| PDGF A chain | 6 x 10⁵ copies/ug RNA | |
| PDGF B chain | 0 | 0 |
| IGF-1 | 5 x 10⁵ copies/ugRNA | |
| EGF | 3 x 10³ copies/ug RNA | |
| HBEGF | 2 x 10⁴ copies/ ug RNA | |
| KGF | 7 x 10⁴ copies/ug RNA | |
| TGF-β1 | 6 x 10⁶ copies/ug RNA | 300 pg/10⁶ cells/day |
| TGF-β3 | 1 x 10⁴ copies/ug RNA | |
| HGF | 2 x 10⁴ copies/ug RNA | 1 ng/10⁶ cells/day |
| IL-1a | 1 x 10₄ copies/ug RNA | Below detection |
| IL-1b | 0 | |
| TNF-α | 1 x 10⁷ copies/ ug RNA | |
| TNF-β | 0 | |
| IL-6 | 7 x 10⁶ copies/ug RNA | 500 pg/10⁶ cells/day |
| IL-8 | 1 x 10⁷ copies/ug RNA | 25 ng/10⁶ cells/day |
| IL-12 | 0 | |
| IL-15 | 0 | |
| NGF | 0 | |
| G-CSF | 1 x 10⁴ copies/ug RNA | 300 pg/10⁶ cells/day |
| Angiopoietin | 1 x 10⁴ copies/ug RNA | |

In addition to the above list of growth factors, the three dimensional tissues are also characterized by the expression of Wnt proteins, wherein the Wnt proteins comprise at least Wnt5a, Wnt7a, and Wnt11. Descriptions of these specific Wnt proteins are further given below.

It is to be understood that additional cell products, including other growth factors, may be produced by the cell cultures such that the scope of the three dimensional tissues is not to be limited by the descriptions above.

### 5.5 Genetically Engineered Cells

Genetically engineered three-dimensional cultured tissue may be prepared as described in U.S. Patent No. 5,785,964. Generally, a genetically-engineered cultured tissue may serve as a gene delivery vehicle for sustained release of growth factors. Cells may be engineered to express an exogenous gene product. In some embodiments, cells that can be genetically engineered include, by way of example and not limitation, fibroblasts, smooth muscle cells, cardiac muscle cells, mesenchymal stem cells, and other cells found in loose connective tissue such as endothelial cells, macrophages, monocytes, adipocytes, pericytes, and reticular cells found in bone marrow.

The cells and tissues may be engineered to express a gene product which may impart a wide variety of functions, including, but not limited to, promoting proliferation of cells in culture, enhancing production of growth factors promoting hair growth, enhancing production of factors promoting vascularization, promoting tissue repair, and promoting tissue regeneration. The gene product may be a peptide or protein, such as an enzyme, hormone, cytokine, a regulatory protein, such as a transcription factor or DNA binding protein, a structural protein, such as a cell surface protein, or the target gene product may be a nucleic acid such as a ribosome or antisense molecule. In some embodiments, the gene product is one or more Wnt proteins, which play a role in differentiation and proliferation of a variety of cells as described below (see, *e.g*., Miller, J.R., 2001, Genome Biology 3:3001.1-3001.15).

In some embodiments, the gene products which provide enhanced properties to the genetically engineered cells, include but are not limited to, gene products which enhance cell growth. Non-limiting examples of such vascular endothelial growth factor (VEGF), hepatocyte growth factor (HGF), fibroblast growth factors (FGF), platelet derived growth factor (PDGF), epidermal growth factor (EGF), transforming growth factor (TGF), and Wnt factors. In some embodiments in which the recombinantly engineered cells are made to express Wnt factors, specific Wnt factors for expression in the cell include, among others, one or more of Wnt5a, Wnt7a, and Wnt11. In other embodiments, the cells and tissues are genetically engineered to express target gene products which result in cell immortalization, *e.g*., oncogenes or telomerese.

In other embodiments, the cells and tissues are genetically engineered to express gene products which provide protective functions *in vitro* such as cyropteservation and anti-desiccation properties, *e.g*., trehalose (U.S. Patent Nos. 4,891,319; 5,290,765; and 5,693,788). The cells and tissues may also be engineered to express gene products which may provide a protective function *in vivo,* such as those that would protect the cells from an inflammatory response and protect against rejection by the host's immune system, such as HLA allelic variants, major histocompatibility epitopes, immunoglobulin and receptor epitopes, moieties of cellular adhesion molecules, cytokines, and chemokines.

There are a number of ways that the gene products may be engineered to be expressed by the cells and tissues. The gene products may be engineered to be expressed constitutively or in a tissue-specific or stimuli-specific manner. In accordance with this aspect, the nucleotide sequences encoding the target gene products may be operably linked to promoter elements which are constitutively active, tissue-specific or induced upon presence of one or more specific stimuli.

In various embodiments, the nucleotide sequences encoding the target gene products are operably linked to regulatory promoter elements that are responsive to shear or radial stress. In such embodiments, the promoter element would be turned on by passing blood flow (shear) as well as the radial stress that is induced as a result of the pulsatile flow of blood through the heart or vessel.

Examples of other regulatory promoter elements include tetracycline responsive elements, nicotine responsive elements, insulin responsive element, glucose responsive elements, interferon responsive elements, glucocorticoid responsive elements estrogen/progesterone responsive elements, retinoic acid responsive elements, viral transactivators, early or late promoter of SV40 adenovirus, the lac system, the trp system, the TAC system, the TRC system, the promoter for 3-phosphoglycerate and the promoters of acid phosphatase. In other embodiments, artificial response elements are constructed, composed of multimers of transcription factor binding sites and hormone-response elements similar to the molecular architecture of naturally-occurring promoters and enhancers (see, *e.g*., Herr and Clarke, 1986, J Cell 45(3): 461-70). Such artificial composite regulatory regions can be designed to respond to any desirable signal and be expressed in particular cell-types depending on the promoter/enhancer binding sites selected. Techniques for constructing the expression systems and genetically engineering cells are found in various reference works, such as Sambrook et al., 2000, Molecular Cloning: A Laboratory Manual, 3rd Ed., Cold Spring Harbor Press, Cold Spring Harbor, NY; Current Protocols in Molecular Biology, Ausubel et al., eds., John Wiley & Sons, 1988, updates to 2005; and Current Protocols in Cell Biology, Bonifacino et al. eds., John Wiley & Sons, 2001, updates to 2005.

### 5.6 Conditioned Medium and Extracellular Matrix

In some embodiments, the compositions comprise conditioned medium made from the three dimensional tissues. As used herein, "conditioned media" refers to culture media in which cells have been cultured and into which the cells have secreted active agent(s) to sufficient levels to possess a desired biological activity or activities. In some embodiments, the "conditioned media" is characterized by a fingerprint or repertoire of cell-produced factors present in the media. The conditioned medium made from three dimensional tissues, such as those described herein, is found to produce various growth factors, including, among others, VEGF and one or more Wnt proteins. Growth factors in the media appear to induce vascularization, recruit stem cells, and promote cell proliferation and differentiation.

The conditioned medium produced by the three dimensional tissues may be used directly or processed in various ways. The medium may be subject to lyophilization for preservation and/or concentration of growth factors. Various biocompatible preservatives, cryoprotectives, and stabilizer agents may be used to preserve activity where required. Non-limiting examples of biocompatible agents include, among others, glycerol, dimethyl sulfoxide, and trehalose. The lyophilizate may also have one or more excipients such as buffers, bulking agents, and tonicity modifiers. The freeze-dried media is reconstituted by addition of a suitable solution or pharmaceutical diluents, as further described below.

In some embodiments, the conditioned media may be processed by precipitating the active components (*e.g*., growth factors) in the media. Precipitation may use various procedures, such as salting out with ammonium sulfate or use of hydrophilic polymers, for example polyethylene glycol.

In other embodiments, the conditioned media is subject to filtration using various selective filters. Processing the conditioned media by filtering is useful in concentrating the growth factors and also removing small molecules and solutes used in the culture medium. Filters with selectivity for specified molecular weights include <5000 Daltons, <10,000 Daltons, and < 15,000 Daltons. Other filters may be used and the processed media assayed for tissue repair and regeneration promoting activity as described herein. Exemplary filters and concentrator system include those based on, among others, hollow fiber filters, filter disks, and filter probes (see, *e.g*., Amicon Stirred Ultrafiltration Cells, Millipore, Billerica, MA, USA).

In still other embodiments, the conditioned medium is subject to chromatography to remove salts, impurities, or to fractionate various components of the medium. Various chromatographic techniques may be employed, such as molecular sieving, ion exchange, reverse phase, and affinity chromatographic techniques. For processing conditioned medium without significant loss ofbioactivity, mild chromatographic media is used. Non-limiting examples include, among others, dextran, agarose, polyacrylamide based separation media (*e.g*., available under various tradenames, such as Sephadex, Sepharose, and Sephacryl).

In other embodiments, the compositions comprise the extracellular matrix (see, *e.g*., US Patent No. 5,830,708). Extracellular matrix produced by the three dimensional tissues also may contain various growth factors, and may be used for the treatments described herein. The extracellular matrix preparation may be used independently of the other compositions or used in combination. Other uses of the extracellular matrix include, among others, as compositions for soft tissue augmentation, such as a substitute or addition to various forms of collagen used in cosmetic surgery and for the repair of skin defects. Depending on the type of collagen desired, appropriate cells, such as stromal cells, are selected for growth in the culture systems. Removal and formulation of the extacellular matrix are described in US Patent No. 5,830,708. Typical methods use detergents to disrupt the cellular membrane and remove cell debris, followed by removal of the extracellular matrix by sonication or enzymatic treatment.

### 5.7 Production and Delivery of Growth Factors

The three dimensional tissues herein produce various cellular growth factors that affect, among others, cell proliferation, differentiation, and recruitment. The three dimensional tissues described herein may be used to deliver the suite or repertoire of growth factors to desired cells, tissues, or organs, or used to produce growth factors for isolation. In some embodiments, the growth factor is delivered by the compositions comprise VEGF, which induces vascular permeability, promotes growth and survival of vascular endothelial cells, and controls hematopoietic stem cell survival. *In vivo,* VEGF promotes angiogenesis and the formation of new blood vessels.

In other embodiments, the growth factors are Wnt factors, which are signaling molecules having roles in a myriad of cellular pathways and cell-cell interaction processes. Wnt signaling has been implicated in tumorigenesis, early mesodermal patterning of the embryo, morphogenesis of the brain and kidneys, regulation of mammary gland proliferation, and Alzheimer's disease.

"Wnt" or "Wnt protein" as used herein refers to a protein with one or more of the following functional activities: (1) binding to Wnt receptors, also referred to as Frizzled proteins, (2) effecting Wnt mediated signaling, (3) modulating phosphorylation of Dishevelled protein and cellular localization of Axin protein (4) modulation of cellular β-catenin levels and corresponding signaling pathway, (5) modulation of TCF/LEF transcription factors, and (6) increasing intracellular calcium and activation of Ca⁺² sensitive proteins (*e.g*., calmodulin dependent kinase). "Modulation" as used in the context of Wnt proteins refers to an increase or decrease in cellular levels, changes in intracellular distribution, and/or changes in functional (*e.g*., enzymatic) activity of the molecule modulated by Wnt.

"Wnt mediated signaling" refers to activation of a cellular signaling pathway initiated by or dependent on interaction of Wnt protein and its cognate receptor protein. As a point of reference, the canonical Wnt signaling pathway involves binding of the Wnt protein to its corresponding cellular receptor, the Frizzled proteins. Receptor activation tranduces a signal by phosphorylation of the protein Dishevelled, which interacts with Axin. This interaction disrupts the formation of a cellular complex comprised of the proteins Axin, Adenomatous Polyposis Coli (APC), and glycogen synthase kinase-3β (GSK-3) that is believed to regulate β-catenin activity by promoting its degradation via a proteosome mediated pathway. Wnt signaling, through its action on Dishevelled and Axin, inhibits degradation of β-catenin, thereby leading to β-catenin accumulation in the cytoplasm and nucleus. β-catenin then interacts with the transcription factor TCF/LEF and promotes its translocation into the nucleus, where the protein complex modulates the transcription of various target genes.

It is to be understood, however, that Wnt signaling is not restricted to the canonical pathway, and that cells may have alternative pathways affected by signal transduction mediated by Wnt. β-catenin has been shown to interact with other types of transcription factors, such as p300/CBP, BRG-1, and LIM domain protein FHL-2. In addition, several non-canonical Wnt signaling pathways have been elucidated that act independently of β-catenin (see, *e.g*., Lustig and Behrens, 2003, J. Cancer Res. Clin. Oncol. 129:199-221; Polakis, P., 2000, Genes Dev. 14:1837-1851). In one noncannonical pathway, Wnt binds to the Frizzled receptor resulting in the activation of heterotrimeric G-proteins and subsequent mobilization of phospholipase C and phosphodiesterase. This activation results in a decrease in cGMP levels, an increase in intracellular Ca⁺², and activation of protein kinase C and other Ca⁺² regulated proteins. A second non-canonical pathway is the planar cell polarity (PCP) pathway that defines polarity in select epithelial tissues, particularly along an axis perpendicular to the apical-basal border. In vertebrates, it may contribute to the differentiation and orientation of inner ear hair cell stereocilia and direct the expansion of mesoderm and neuroectoderm during gastrulation (Dabdoub and Kelley, 2005, J. Neurobiol. 64(4):446-57). It is thought that activation of the PCP pathway occurs by Wnt binding to Frizzled, which activates Dishevelled. Dishevelled then recruits RhoA/Rac, which ultimately leads to JNK (c-jun NH2-terminal kinase) pathway activation. A major target of the JNK pathway appears to be the AP-1 (activator protein-1) transcription factor.

"Wnt" or "Wnt proteins" are also characterized structurally by their sequence similarity or identity to mouse Wnt-1 and Wingless in *Drosophila.* As used herein "percentage of sequence identity" and "percentage homology" are used interchangeably herein to refer to comparisons among polynucleotides and polypeptides, and are determined by comparing two optimally aligned sequences over a comparison window, wherein the portion of the polynucleotide or polypeptide sequence in the comparison window may comprise additions or deletions (*i.e*., gaps) as compared to the reference sequence (which does not comprise additions or deletions) for optimal alignment of the two sequences. The percentage may be calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Alternatively, the percentage may be calculated by determining the number of positions at which either the identical nucleic acid base or amino acid residue occurs in both sequences or a nucleic acid base or amino acid residue is aligned with a gap to yield the number of matched positions, dividing the number of matched positions by the total number of positions in the window of comparison and multiplying the result by 100 to yield the percentage of sequence identity. Those of skill in the art appreciate that there are many established algorithms available to align two sequences. Optimal alignment of sequences for comparison can be conducted, *e.g*., by the local homology algorithm of Smith and Waterman, 1981, Adv. Appl. Math. 2:482, by the homology alignment algorithm of Needleman and Wunsch, 1970, J. Mol. Biol. 48:443, by the search for similarity method of Pearson and Lipman, 1988, Proc. Natl. Acad. Sci. USA 85:2444, by computerized implementations of these algorithms (GAP, BESTFIT, FASTA, and TPASTA in the GCG Wisconsin Software Package), or by visual inspection (see generally, Current Protocols in Molecular Biology, (F. M. Ausubel et al., eds.), John Wiley & Sons, Inc., 1995 Supplement). Examples of algorithms that are suitable for determining percent sequence identity and sequence similarity are the BLAST and BLAST 2.0 algorithms, which are described in Altschul et al., 1990, J. Mol. Biol. 215: 403-410 and Altschul et al., 1977, Nucleic Acids Res. 3389-3402, respectively. Software for performing BLAST analyses is publicly available through the National Center for Biotechnology Information website. This algorithm involves first identifying high scoring sequence pairs (HSPs) by identifying short words of length W in the query sequence, which either match or satisfy some positive-valued threshold score T when aligned with a word of the same length in a database sequence. T is referred to as, the neighborhood word score threshold (Altschul et al, supra). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff, 1989, Proc. Natl. Acad. Sci. USA 89:10915).

While all of the above mentioned algorithms and programs are suitable for a determination of sequence alignment and % sequence identity, for determination of % sequence identity in some embodiments the BESTFIT or GAP programs in the GCG Wisconsin Software package (Accelrys, Madison WI), is used with the default parameters provided.

Of relevance to the present disclosure are Wnt proteins expressed in mammals, such as rodents, felines, canines, ungulates, and primates. For instance, human Wnt proteins that have been identified share 27% to 83% amino-acid sequence identity. Additional structural characteristics of Wnt protein are a conserved pattern of about 23 or 24 cysteine residues, a hydrophobic signal sequence, and a conserved asparagine linked oligosaccharide modification sequence. In some embodiments, Wnt proteins are also lipid modified, such as with a palmitoyl group (Wilkert et al., 2003, Nature 423(6938):448-52). Exemplary Wnt proteins and its corresponding genes expressed in mammals include, among others, Wnt 1, Wnt 2, Wnt 2B, Wnt 3, Wnt3A, Wnt4, Wnt 4B, Wnt5A, Wnt 5B, Wnt 6, Wnt 7A, Wnt 7B, Wnt8A, Wnt8B, Wnt9A, Wnt9B, Wnt10A Wnt11, and Wnt 16. Other identified forms of Wnt, such as Wnt12, Wnt13, Wnt14, and Wnt 15, appear to fall within the proteins described for Wnt 1-11 and 16. Protein and amino acid sequences of each of the mammalian Wnt proteins are available in databases such as SwissPro and Genbank (see, *e.g*., US Published Application No. 20040248803. Within the scope of "Wnt" and "Wnt proteins" are protein fragments, variants, and mutants of the identified Wnt proteins, where the fragments, variants, and mutants have the functional activities characteristic of the family of Wnt proteins.

In the embodiments herein, the "suite", "repertoire", "signature" or "fingerprint" of Wnt factors elaborated by the three dimensional tissues may be used to promote tissue repair and tissue regeneration. Wnt factors produced by the three dimensional tissues comprise at least Wnt5a, Wnt7a, and Wnt11, which defines a characteristic or signature of the Wnt proteins present in the conditioned media. As used herein, Wnt5a refers to a Wnt protein with the functional activities described above and sequence similarity to human Wnt protein with the amino acid sequence in NCBI Accession Nos. AAH74783 (gI:50959709) or AAA16842 (gI:348918) (see also, Danielson et al., 1995, J. Biol. Chem. 270(52):31225-34). Wnt7a refers to a Wnt protein with the functional properties of the Wnt proteins described above and sequence similarity to human Wnt protein with the amino acid sequence in NCBI Accession Nos. BAA82509 (gI:5509901); AAC51319.1 (gI:2105100); and 000755 (gI:2501663) (see also, Ikegawa et al., 1996, Cytogenet Cell Genet. 74(1-2):149-52; Bui et al., 1997, Gene 189(1):25-9). Wnt11 refers to a Wnt protein with the functional activities described above and sequence similarity to human Wnt protein with the amino acid sequence in NCBI Accession Nos. BAB72099 (gI:17026012); CAA74159 (gI:3850708); and CAA73223.1 (gI:3850706) (see also, Kirikoshi et al., 2001, Int. J. Mol. Med. 8(6):651-6); Lako et al., 1998, Gene 219(1-2):101-10). As used herein in the context the specific Wnt proteins, "sequence similarity" refers to an amino acid sequence identity of at least about 80% or more, at least about 90% or more, at least about 95% or more, or at least about 98% or more when compared to the reference sequence. For instance, human Wnt7a displays about 97% amino acid sequence identity to murine Wnt7a while the amino acid sequence of human Wnt7a displays about 64% amino acid identity to human Wnt5a (Bui et al., *supra*).

In other embodiments, isolated Wnt proteins are used alone to promote tissue repair and regeneration or as a supplement to the conditioned media produced from the three dimensional tissue. As noted above, a number of different Wnt proteins have been determined to be produced in the three dimensional tissues and may be isolated by the methods described herein. Isolated Wnt proteins that may be useful for the methods herein include Wnt5, Wnt7 and Wnt is 11a, as described above.

The suite of Wnt proteins elaborated by the cell culture or the individual Wnt proteins may be isolated by various techniques available to the skilled artisan. Because of the lipid modification of Wnt proteins, purification typically uses detergents to solubilize and maintain the activity of Wnt proteins. These methods are described in Willert et al., 2003, Nature 423(6938):448-52 and U.S. Published Application 20040248803. The Wnt proteins made in the three dimensional tissue may be solubilized with non-anionic detergents or zwitterionic detergents at a concentration of from about 0.25% to about 2.5%, at a concentration of from about 0.5% to 1.5%, or at a concentration of about 1%. In some embodiments, suitable non-anionic detergents for solubilizing the Wnts are members of detergents available under the tradename Triton, including Triton X-15, Triton X-35, Triton X45, Triton X-100, Triton X-102, Triton X-114, and Triton X-165. In some embodiments, solubilization may be combined with other purification techniques to obtain isolated or enriched preparations of Wnt. These include other art known techniques such as reverse phase chromatography high performance liquid chromatography, ion exchange chromatography, gel electrophoresis, affinity chromatography (*e.g*., dye ligand with Cibaron Blue) of solubilized Wnt proteins. The actual conditions used to isolate the Wnt proteins will depend, in part, on factors such as net charge, hydrophobicity, hydrophilicity, molecular weight, etc., and will be apparent to those having skill in the art, as described in U.S. Published Application No. 20040248803.

In other embodiments, antibodies to identified Wnt proteins may be used *en masse* to isolate the suite of Wnt proteins produced by the three dimensional tissue. In other embodiments, an antibody directed to a common epitope expressed in different Wnt proteins may be used to isolated multiple Wnt proteins. In still other embodiments, antibodies to specific Wnt proteins (*e.g*., Wnt5a, Wnt7a, and Wnt11) may be used to isolate a single type of Wnt protein produced by the cultures. Antibodies may be immobilized on a column or to a solid substrate (*e.g*., magnetic beads, agarose beads, etc.) to isolate the Wnt proteins or alternatively may be precipitated by agents such as Staph A protein or other antibody binding agents. Procedures for antibody based purification are described in many reference works, such as Ausubel, Current Methods in Molecular Biolgy, JohnWiley & Sons, updates to 2005; Harlow and Lane, 1988, Antibodies: A Laboratory Manual, Cold Spring Harbor Laboratory Press, Cold Spring Harbor, NY; Scopes, 1984, Protein Purification: Principles and Practice, Springer Verlag New York, Inc., N.Y.; and Livingstone, 1974, Methods In Enzymology: Immunoaffinity Chromatography of Proteins 34:723 731.

In other embodiments, the Wnt proteins may be made by recombinant methods using methods well known in the art, for example, as described in U.S. Published Application No. 20040248803.

### 5.8 Pharmaceutical Compositions

The compositions of three dimensional tissues may be used directly for administration or prepared with pharmaceutically acceptable vehicles. As used herein, a "pharmaceutically acceptable vehicle" refers to a carrier, excipient or diluent for administering the compositions. These may include cell media components typically used in the art of cell culture. Compositions may be suspended in serum free culture medium, basal culture media, complex culture media, and balanced salt solutions. In other embodiments, the media may contain pharmaceutically acceptable additives, such as vitamins, inorganic salts, amino acids, carbon sources, fatty acids, buffers, and serum. Non limiting examples of media and diluents include phosphate buffered saline, Hanks Balanced Salt Solution, Earles salts, Modified Eagles Medium, Dulbecco's Modified Eagles Medium, RPMI medium, Iscoves medium, and Leibovitz L-15. Resuspension or replacement with fresh cell medium may be done shortly before administration of the three dimensional tissues.

In other embodiments, the compositions of cells are cryopreserved preparations, which are thawed prior to use. Pharmaceutically acceptable cryopreservatives include, among others, glycerol, saccharides, polyols, methylcellulose, and dimethyl sulfoxide. Saccharide agents include monosaccharides, disaccharides, and other oligosaccharides with glass transition temperature of the maximally freeze-concentrated solution (Tg) that is at least-60,-50,-40,-30,-20,-10, or 0°C. An exemplary saccharide for use in cryopreservation is trehalose. Cryopreservation is used not only for storage purposes but may also be carried out to increase the production of growth factors (US Patent No. 6,291,240)

In some embodiments, the three dimensional tissues are treated to kill the cells prior to use. In some embodiments, the extracellular matrix deposited on the scaffolds may be collected and processed for administration for various medical and cosmetic applications (see US Patent Nos. 5,830,708 and 6,280,284. In other embodiments, the three dimensional tissue in which the cells have been killed, and thus lack viable cells, are administered to promote tissue repair and regeneration.

In other embodiments, the three dimensional tissue may be concentrated and washed with a pharmaceutically acceptable medium for administration. Various techniques for concentrating the compositions are available in the art, such as centrifugation or filtering. Exemplary techniques include as non-limiting examples, dextran sedimentation and differential centrifugation. Formulation of the three dimensional tissues may involve adjusting the ionic strength of the suspension to isotonicity (i.e., about 0.1 to 0.2) and to physiological pH (*i.e*., pH 6.8 to 7.5). The formulation may also contain lubricants or other excipients to aid in administration or stability of the cell suspension. These include, among others, saccharides (*e.g*., maltose) and organic polymers, such as polyethylene glycol and hyaluronic acid. Additional details for preparation of various formulations are described in US Patent Publication No. 2002/0038152.

In still other embodiments, the compositions further comprise image contrast agents for imaging the compositions *in vivo.* Various types of imaging techniques and corresponding imaging enhancing media include, but are not limited to, ultrasounds media, magnetic resonance contrast media, computed axial tomography contrast media, X-ray diagnostic contrast media, and positron emission tomography contrast media. Non-limiting examples of contrast agents include, among others, gadoliniumn complexes, barium, iodine, encapsulated microbubblcs, and polymeric microparticles (*e.g*., PLGA). These can be used to determine the location of the administered compositions and in some instances the integrity of the compositions *in vivo* (see, *e.g.,* Ultrasound Contrast Agents: Basic Principles and Clinical Applications, 2nd Ed., BB Goldberg ed., Taylor & Francis Group, 2001; Lathia et al, 2004, Pharmaceutical Engineering 24(1):1-8; "Contrast Agents I: Magnetic Resonance Imaging," in Topics in Current Chemistry, Vol 221, Krause ed., Spinger-Verlag, 2002) As will be apparent to the skilled artisan, some forms of the three dimensional scaffolds, such as microparticles, may have properties that allow the three dimensional tissue to act itself as an image contrast agent and thus to be detectable using the corresponding imaging technique.

### 5.9 Administration

The compositions may be administered at specific sites in or on tissues or organs. The cell culture compositions are administered in an amount effective to treat the specified condition or disorder. The compositions may be administered by various methods know to the skilled artisan. The compositions and medicaments are administered by injection into ischemic heart, such as with a hypodermic needle. The size (*i.e*., gauge) of the hypodermic needle will depend on factors such as the type of composition, the amount being injected, the spatial location for depositing the composition. Typical gauges for injection are available from 12 to 25 gauge of various lengths.

In other embodiments, the compositions are administered using a catheter. The catheter may be a flexible, rigid, or semi rigid tube or conduit positioned at the site for deposition of the cultured tissues. The catheter may be made of various materials, non-limiting example of which include, among others, plastic, metal, and silicon. Where the compositions comprise cords or sutures, they may be administered by injection or catheter, but may also be introduced into tissue sites by methods typically used for suturing tissues, *e.g*., using an attached suturing needle. The cord or suture is threaded into the tissue and then left in place by detaching the suturing needle.

In still other embodiments, an incision is made in the tissue or organ, and the compositions applied into the incision site. The compositions may be held in place by suturing the tissue or organ at the site of the incision to cover and contain the compositions. Placement of the compositions may be done during surgery to repair ischemic heart damaged tissue, which may enhance repair of the surgical damage as well as the tissue damaged by a disorder or disease.

In some embodiments, the administration of the compositions may be guided by various medical imaging techniques, including, but not limited to, ultrasound, fiber optic, magnetic resonance imaging, or computer assisted tomography. As noted above, the three dimensional framework may have contrast agents to assist in imaging of the compositions as it is administered into the subject.

The dosages for administration will take into consideration various factors such as the nature of the condition being treated, the type of tissue or organ, the amount that the tissue or organ can accommodate, degradation properties of the three dimensional scaffold *in vivo*, duration of cell activity following administration, and the level of growth factors produced. Three dimensional frameworks that degrade at a faster rate may be administered at a higher frequency without significant accumulation of the framework material in the tissue or organ while materials with slower degradation rates may be administered with lower frequency to limit the amount of undegraded material present in the injected site. The frequency of administration may also be adjusted for elimination of the framework material by bodily mechanisms, such as through systemic circulation and the lymphatic system.

In various embodiments, the compositions may be administered once per day, about twice per week, about once per week, about once every two weeks, about once every month, or about once per six months, or more or less depending, at least in part, on the factors discussed above. The compositions may be administered at different sites concurrently or sequentially. When administered at different sites, the administrations may be to a localized area. The spatial density of administration in a localized area may depend on the extent of the tissue or organ being treated, such as volume and surface area as well as depth of the treated site. In some embodiments, when treating a layer of tissue, the compositions may be administered about 1 per cm², about 2 per cm², about 4 per cm², or 6 per cm² or more as necessary. In still other embodiments, the compositions may be administered in a volume of tissue, for example, 1 per cm³, 2 per cm³, 4 per cm³ or 6 per cm³, or more as necessary to provide a therapeutic benefit. When administering within a tissue or organ, injection may be done at the same depth or at different depths. In some embodiments, the compositions are administered into a body cavity, either naturally occurring or induced by injury, disease, surgery, or other conditions described above.

### 5.10 Uses of Three Dimensional Tissues

The compositions and medicaments according to claim 1 and 2 comprising the three dimensional tissues may be used for a variety of therapies. In some embodiments, the compositions and medicaments are used to treat (*e.g*., repair or regenerate) ischemic heart.

In some embodiments, the compositions are used to treat acute tissue damage. As used herein, "acute damage" refers to heart damage or wounds caused by, acute ischemia and reperfusion injury. Traumatic force injury includes, among others, surgical procedures and blunt force trauma (*e.g*., gun shot wounds, knife wounds, etc.). The compositions and medicaments may be injected into the affected heart tissues to promote vascularization, repair, and regeneration of such damaged tissues.

In other embodiments, the compositions are used to treat chronic tissue damage. As used herein, "chronic tissue damage" refers to heart tissue damage resulting from persistent or repeated insults to a tissue, typically showing manifestations of persistent or chronic inflammatory reaction or unhealed or improper healing of tissue. Chronic tissue damage may also be characterized by the presence tissue remodeling, such as fibrosis, known as scarring, originating from the repeated insult. Other forms of tissue remodeling in chronic tissue damage include, among others, thickening of tissue arising from compensatory changes to reduced tissue function, or tissue thinning where cytopathic effects result in continual loss of cells without compensatory cell renewal. Some chronic tissue damage may show tissue thinning during the early stages of damage followed by tissue thickening arising from repeated scarring and/or compensation for reduced tissue function. Chronic tissue damage may arise in many different contexts, such as repeated exposure to irritants or toxic chemicals, persistent or repeated ischemic events (*e.g*., chronic ischemia, micro-strokes), chronic infections, and persistent disease condition (*e.g*., autoimmune disease, ulcers, atherosclerosis, congenital defects, etc.).

In this invention, the tissue damage comprises ischemic damage to the heart. As used herein, "ischemic tissue" refers to tissues that have been deprived of blood or oxygen supply, thereby resulting in injury to cells and tissues. On the cellular level, ischemia is any process in which there is a lack of sufficient blood flow to a portion of the tissue, thereby initiating an ischemic cascade, leading to the death of cells. For instance, myocardial ischemia is a condition in which oxygen deprivation to the heart muscle is accompanied by inadequate removal of metabolites because of reduced blood flow or perfusion. Myocardial ischemia can occur as a result of increased myocardial oxygen demand, reduced myocardial oxygen supply, or both. Myocardial ischemia may be caused by reduction of oxygen supply secondary to increased coronary vascular tone (*i.e*., coronary vasospasm) or by marked reduction or cessation of coronary flow as a result of platelet aggregates or thrombi.

"Acute ischemia" refers to an abrupt or sudden disruption in blood flow to tissues. For instance, acute ischemia in the heart, also known as myocardial infarction, is generally caused by a rapid occlusion of the coronary arteries, such as that arising from ruptured proximal arteriosclerotic plaque, acute thrombosis on preexisting atherosclerotic disease, an embolism from the heart, aorta, or other large blood vessel, or a dissected aneurysm. In embodiments in which acute ischemia is diagnosed, the compositions may be applied onto or into the area of ischemically damaged tissue to promote vascularization, increase blood flow to the muscles and promote regeneration of heart tissue.

"Chronic ischemia" typically refers to disruption in blood flow to tissues by gradual enlargement of an atheromatous plaque that reduces blood flow to the affected downstream tissue. As cells die and the tissue becomes damaged, remodeling may occur, such as tissue thinning from cell death, and tissue thickening and disorganization from scarring events arising from cellular response to the damage.

The compositions and medicaments are used to treat an ischemically damaged heart tissue, various forms of which include, among others, acute myocardial ischemia, chronic myocardial ischemia, and congestive heart failure. Other disorders of the heart, such as cardiomyopathy, may also be treated with the compositions and medicaments described herein. As noted above, cardiovascular ischemia may be caused by a rupture of an atherosclerotic plaque in a coronary artery, leading to formation of thrombus, which can occlude or obstruct a coronary artery, thereby depriving the downstream heart muscle of oxygen. Necrosis resulting from the ischemia is commonly called an infarct.

Chronic ischemia in the heart is believed to occur by gradual enlargement of an atheromatous plaque that reduces blood flow to the heart. As the heart weakens, remodeling occurs, typically in the ventricles, and the heart enlarges and becomes rounder. The heart also undergoes changes at the cell level characterized by cell apoptosis, resulting in a less distensible heart and a weakening of the heart muscle over time.

"Congestive heart failure" refers to impaired cardiac function in which the heart fails to maintain adequate circulation of blood, and in some embodiments, is the end result of damage from chronic ischemia. The most severe form of congestive heart failure leads to pulmonary edema, which develops when this impairment causes an increase in lung fluid secondary to leakage from pulmonary capillaries into the interstitium and alveoli of the lung. In some embodiments, heart function in congestive heart failure is expressed as an imbalance in the degree of end-diastolic fiber stretch proportional to the systolic mechanical work expended in an ensuing contraction (also known as the Frank-Starling principle). Various parts of the heart may be affected, including left ventricle and right ventricle.

"Cardiac myopathy" is typically defined by any structural or functional abnormality of the ventricular myocardium, except for congenital developmental defects, valvular disease; systemic or pulmonary vascular disease; isolated pericardial, nodal, or conduction system disease; or epicardial coronary artery disease; unless chronic diffuse myocardial dysfunction is present. Based upon clinical indications, the disorder may be diagnosed as dilated congestive, hypertrophic, or restrictive cardiomyopathy. Dilated congestive cardiomyopath is generally characterized chronic myocardial fibrosis with diffuse loss of myocytes. Without being limited by theory, the underlying pathologic process is believed to start with an acute myocarditic phase, which may have viral causes, followed by a variable latent phase, then a phase of chronic fibrosis and death of myocardial myocytes due to an autoimmune reaction to virus-altered myocytes. Whatever the cause of the disorder, it leads to dilation, thinning, and compensatory hypertrophy of the remaining myocardium interspersed with fibrosis. Functionally, there is impaired ventricular systolic function reflected by a low ejection fraction (EF). Hypertrophic cardiomyopathy is characterized by marked ventricular hypertrophy with diastolic dysfunction. At the cellular level, the cardiac muscle is abnormal with cellular and myofibrillar disarray. The most common asymmetric form of hypertrophic cardiomyopathy displays marked hypertrophy and thickening of the upper interventricular septum below the aortic valve. The hypertrophy results in a stiff, noncompliant chamber that resists diastolic filling, leading to elevated end-diastolic pressure, which raises pulmonary venous pressure. Restrictive cardiomyepathy is characterized by rigid, noncompliant ventricular walls that resist diastolic filling of one or both ventricles, most commonly the left. This less frequent form of cardiacmyopathy has different causes, often being associated with other disorders or conditions, such as Gaucher's Disease, Loffler's Disease, amyloidosis, and endorcardial fibrosis. Physiology of the heart shows endocardial thickening or myocardial infiltration with loss of myocytes, compensatory hypertrophy, and fibrosis, all of which may lead to atrioventricular valve malfunction. Functionally, the heart shows diastolic dysfunction with a rigid, noncompliant chamber with a high filling pressure. Systolic function may deteriorate if compensatory hypertrophy is inadequate in cases of infiltrated or fibrosed chambers.

Various criteria may be used to diagnose disorders of the heart and are provided in various reference works (see, *e.g*., Dec et al., HeartFailure: A Comprehensive Guide To Diagnosis And Treatment, Marcel Dekker, 2004; The Merck Manual of Diagnosis and Therapy, 17th Ed (M. H. Beer and R. Berkow eds.), John Wiley & Sons, 1999). For example, electrocardiogram (ECG) may be used to identify patients suspected of having myocardial infarction. Transmural infarcts involve the whole thickness of myocardium from epicardium to endocardium and are usually characterized by an initial ECG with abnormal deep Q waves and elevated ST segments in leads subtending the area of damage, or characterized by an abnormal ECG with elevated or depressed ST segments and deeply inverted T waves without abnormal Q waves. Nontransmural or subendocardial infarcts do not extend through the ventricular wall and cause only ST segment and T-wave abnormalities. Subendocardial infarcts usually involve the inner third of the myocardium where wall tension is highest and myocardial blood flow is most vulnerable to circulatory changes. Because the depth of necrosis arising from the acute ischemic event cannot be precisely determine clinically, infarcts are generally classified by ECG as Q wave and nun-Q wave.

Other diagnostic methods useful for detecting cardiovascular ischemia include, among others, perfusion imaging using thallium (²⁰¹Tl) or technetium (^{99m}Tc) myocardial perfusion agents, echocardiography, and/or cardiac catheterization. Echocardiography allows evaluation of wall motion, presence of ventricular thrombus, papillary muscle rupture, rupture of the ventricular septum, ventricular function, and presence of intracavitary thrombus. When the diagnosis of myocardial ischemia is uncertain, presence of left ventricle wall motion abnormality by echocardiography establishes the presence of myocardial damage arising from a recent or remote mycocardial infarction. In cardiac catheterization, an imaging contrast medium is injected through the catheter to examine for narrowing or blockages present in the coronary arteries, measure functioning of valves and heart muscle, and/or obtain a biopsy for further analysis.

For treating damage to heart tissue, the compositions and medicaments may be applied to various heart tissues, including, epicardium, myocardium, and/or endocardium. Because the damage may affect different portions of the heart, administration may be into the damaged tissue and/or in surrounding tissues. For instance, left ventricular failure characteristically develops in coronary artery disease, hypertension, and most forms of cardiomyopathy. Right ventricular failure is commonly caused by, among others, prior left ventricular failure, or right ventricular infarction. Thus in some embodiments, where applicable, administration may be to the left ventricular myocardium or to both the left and right ventricular myocardium.

Without being bound by theory, application of compositions and medicaments according to claim 1 or 2 to an ischemic tissue promotes various biological activities involved in the healing of ischemic tissue. Among such activities is the reduction or prevention of the remodeling of ischemic tissue. By "remodeling" herein is meant, the presence of one or more of the following: (1) a progressive thinning of the ischemic tissue, (2) a decrease in the number or blood vessels supplying the ischemic tissue, and/or (3) a blockage in one or more of the blood vessels supplying the ischemic tissue, and if the ischemic tissue comprises muscle tissue, (4) a decrease in the contractibility of the muscle tissue. Untreated, remodeling typically results in a weakening of the ischemic tissue such that it can no longer perform at the same level as the corresponding healthy tissue.

Accordingly, in some embodiments, application of the compositions and medicaments of this invention to an ischemic tissue increases the number of blood vessels present in the ischemic tissue, as measured using laser Doppler imaging (see, *e.g*., Newton et al., 2002, J Foot Ankle Surg. 41 (4):233-7). In some embodiments, the number of blood vessels increases 1%, 2%, 5%; in other embodiments, the number of blood vessels increases 10%, 15%, 20%, even as much as 25%, 30%, 40%, 50%; in some embodiments, the number of blood vessels increase even more, with intermediate values permissible.

In some embodiments, application of the compositions and medicaments of this invention to an ischemic heart tissue increases the ejection fraction. In a healthy heart, the ejection fraction is about 65 to 95 percent. In a heart comprising ischemic tissue, the ejection fraction is, in some embodiments, about 20 - 40 percent. Accordingly, in some embodiments, treatment with the compositions and medicaments of this invention results in a 0.5 to 1 percent absolute improvement in the ejection fraction as compared to the ejection fraction prior to treatment. In other embodiments, treatment with the compositions and medicaments of this invention results in an absolute improvement in the ejection fraction of more than 1 percent. In some embodiments, treatment results in an absolute improvement in the ejection fraction of 1.5%, 2%, 3%, 4%, 5%, 6%, 7%, 8%, even as much as 9% or 10%, as compared to the ejection fraction prior to treatment. For example, if the ejection fraction prior to treatment was 40%, then following treatment ejection fractions between 41 % to 59% are observed in these embodiments. In still other embodiments, treatment with the cultured three-dimensional tissue results in an improvement in the ejection fraction greater than 10% as compared to the ejection fraction prior to treatment.

In some embodiments, application of the compositions and medicaments of this invention to an ischemic heart tissue increases one or more of cardiac output (CO), left ventricular end diastolic volume index (LVEDVI), left ventricular end systolic volume index (LVESVI), and systolic wall thickening (SWT). These parameters are measured by art-standard clinical procedures, including, for example, nuclear scans, such as radionuclide ventriculography (RNV) or multiple gated acquisition (MUGA), and X-rays.

In some embodiments, application of the compositions and medicaments of this invention to an ischemic heart tissue causes a demonstrable improvement in the blood level of one or more protein markers used clinically as indicia of heart injury, such as creatine kinase (CK), serum glutamic oxalacetic transaminase (SGOT), lactic dehydrogenase (LDH) (see, *e.g*., U.S. Publication 2005/0142613), troponin I and troponin T can be used to diagnose heart muscle injury (see, *e.g.,* U.S. Publication 2005/0021234). In yet other embodiments, alterations affecting the N-terminus of albumin can be measured (see, *e.g*., U.S. Publications 2005/0142613, 2005/0021234, and 2005/0004485).

The compositions and medicaments described herein can be used in combination with conventional treatments, such as the administration of various pharmaceutical agents and surgical procedures. For example, in some embodiments, the compositions and/or medicaments are administered with one or more of the medications used to treat heart failure. Medications suitable for use in the methods described herein include angiotensin-converting enzyme (ACE) inhibitors (*e.g*., enalapril (Vasotec), lisinopril (Prinivil, Zestril) and captopril (Capoten)), angiotensin II (A-II) receptor blockers (e.g., losartan (Cozaar) and valsartan (Diovan)), diuretics (e.g., bumetanide (Bumex), furosemide (Lasix, Fumide), and spironolactone (Aldactone)), digoxin (Lanoxin), beta blockers, and nesiritide (Natrecor) can be used.

In other embodiments, the compositions and medicaments can be administered during a surgical procedure, such as angioplasty, single CABG, and/or multiple CABG. Additionally, the compositions and medicaments can be used with therapeutic devices used to treat heart disease including heart pumps, endovascular stents, endovascular stent grafts, left ventricular assist devices (LVADs), biventricular cardiac pacemakers, artificial hearts, and enhanced external counterpulsation (EECP).

The compositions and/or medicaments may be are used to treat chronic liver damage. Distinguishable disorders of the liver including, among others, cirrhosis, fibrosis, and primary biliary cirrhosis. For treating chronic liver damage, the composition may be administered by injection or catheter into the liver, either in the damaged and/or undamaged areas to repair damage and/or enhance regeneration of hepatic tissue. The compositions may be used alone or in combination with other treatments, such as anti-inflammatory agents and anti-viral agents.

The compositions may be used to treat damage to bone marrow and impairment of hematopoiesis. Damage to hematopoietic system typically arise in the context of hematopoietic stem cell transplantation (HSCT) used to treat various cell proliferative disorders of the lymphoid and myeloid systems. The compositions may be administered following cell ablative therapy to promote repair and regeneration of the hematopoietic system. Typical cell ablative therapy used for HSCT include, among others, cytotoxic agents (*e.g*., cyclophosphamide, busulfan, cytosine arabinoside, etc.), radiation, and combinations thereof.

The compositions may be used to promote repair and healing of anastomosis. An anastomosis refers to an operative union between two hollow or tubular structures or a connection between two tissue structures by way of surgery, disease, or trauma. For instance, a pathological anastomosis is a fistula, which is an abnormal connection between an organ, blood vessel, or intestine and another structure. Exemplary surgical anastomoses include vascular graft during a coronary artery bypass graft and the creation of an opening between the bowel and abdominal skin in a colostomy. Examples in the vascular field include, but are not limited to, precapillary (between arterioles), Riolan's (intermesenteric arterial communication between the superior and inferior mesenteric arteries), portal systemic (superior-middle inferior rectal veins; portal vein -inferior vena cava), termino-terminal (artery to vein), and cavopulmonary (treating cyanotic heart disease by anastomosing the right pulmonary artery to the superior vena cava).

Where repair and healing of the anastomotic site is desirable, the compositions may be applied in the region where the tissues meet. -Three dimensional tissues formed on braided sutures may be used to join separated tissues, thereby promoting repairing and healing at the anastomotic site. Non-limiting examples where three dimensional tissue suitable as suture material can be applied include, among others, during organ transplantation procedures, such as for heart, kidney, liver, and lung transplantation.

Further enhancement in repair may be possible by wrapping a site treated with the compositions described herein with patches of three dimensional tissues, such as that available under the tradename Dermagraft® (Smith & Nephew, Indianapolis, IN, USA).

In still other embodiments, the compositions and medicaments of the invention are used to deliver a suite or repertoire of growth factors to a damaged tissue. As described herein, the three dimensional cell tissues produce a growth factors such as VEGF and one or more Wnt proteins. These growth factors may induce and support vascularization, tissue repair, and tissue regeneration. Thus, the compositions, and medicaments of this invention may be used to deliver these growth factors to a desired site (*e.g*., damaged or diseased tissue). In some embodiments, the compositions are used to administer Wnt factors to treat various disorders and conditions, including those described herein.

### 5.11 Kits

Further provided herein are kits comprising the compositions of claim 1 such as in the form of microparticles of in vitro-cultured three dimensional tissues, conditioned media, or cryopreserved formulations thereof. Compositions may be provided in single use disposable containers, such as cell culture bags containing living or cryopreserved three dimensional tissues. Kits may also include devices for administering the three dimensional tissues, such as syringes or surgical needle attached to a suture. In other embodiments, the kits also include instructions for use of the kit components. Various formats include, among others, print medium, computer readable forms (*e.g*., compact disc, magnetic tape, flash memory, etc.), and/or videotape.

Unless defined otherwise, all technical and scientific terms used herein have the same meaning as commonly understood by one of ordinary skill in the art to which this invention belongs. Unless mentioned otherwise the techniques employed or contemplated herein are standard methodologies well known to one of ordinary skill in the art. The materials, methods and examples are illustrative only and not limiting.

### 6. EXAMPLES

### 6.1 Example 1 Culturing of stromal cells on micro-beads

A study was conducted to determine the time required for cell cultures to develop on cultured beads and to be able to pass through a 25 gauge needle. In this experiment, 200 mg of pre-wetted Alkermes® beads (Medisorb®) and smooth muscle cells ("SMCs"; 1 x 10⁶/ml) were added to a bioreactor simultaneously, kept in suspension for two minutes, and allowed to settle down on the bottom of the bioreactor for thirty minutes to enhance the attachment of the cells to the beads. The seeding process (suspension/static) was repeated 6 more times for a total of three hours, after which the cultures were kept in continuous suspension. Samples were collected at various time points and subjected to an MTT assay to assess cell viability. VEGF and DNA analyses were also used to assess cell attachment, viability, and growth.

In order to determine whether storage conditions may have any effect on cells cultured in presence of micro-beads, the cells (20ul) cultured in presence of micro-beads were placed in each of two Eppendorf tubes and shipped to University of Arizona via the "Same Business Day" shipment. Upon arrival, the cultured micro-beads were transferred to the 24-well a non-tissue culture treated plate and agitated with very slow motion in an incubator and then examined for cell viability using an MTT assay.

Cell viability and secreted factors following the passage of minimally invasive constructs through a 24 gauge needle were evaluated. For these experiments, cultured micro-beads were placed in 1 ml of 10% FBS media for 24 hours after which they were subjected to VEGF and MTT assays. Four other treatment groups were also included in this experiment to compare the cell activity. Growth factor expression was examined both by estimation of mRNA by polymerase chain reaction (PCR) methods and estimation of the free protein by enzyme-linked immunosorption assay (ELISA).

Two microparticles were evaluated as scaffolds to form the three dimensional injectable compositions: a non porous PLGA microsphere, which has been cultured for up to 10 weeks with no signs of degradation, and a porous (spray-dried) microsphere. The porous microspheres degraded completely within 2 weeks of culture.

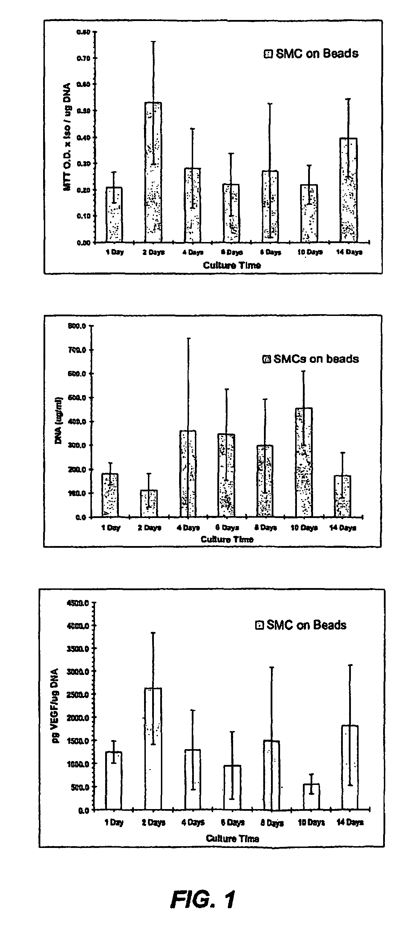

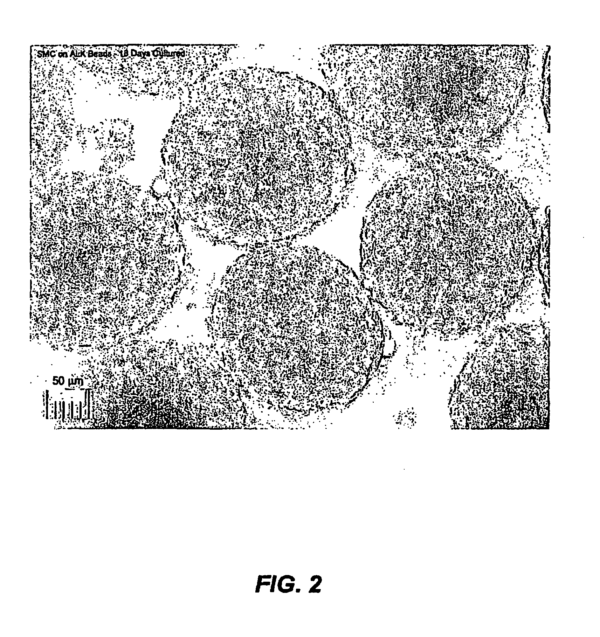

Referring to the MTT, DNA and VEGF assay results of FIG. 1, smooth muscle cells attached, remained viable, and grew on the Alkermes® micro-beads. Light microscopy of the samples confirmed the attachment and growth of the SMCs on Alkermes® beads after 18 days (FIG. 2). From this figure, it can be observed that cultured beads were translucent spheres with a contour of cells surrounding the bead(s) inside. These spheres became more translucent as the beads inside degraded. Dermal fibroblasts (DmFb) cultured on Alkermes® non-porous PLGA microspheres were also found to attach and proliferate on and between the microspheres.

Additionally, it was observed that it would take approximately 21 days to culture beads that could pass through 25 gauge non-removable Becton Dickinson needles. Overall, this study verified that the previously developed seeding method was effective and that the time to harvest the cultured beads was successfully determined. Following passage of cultured beads through the needle, the micro beads retained their original, spherical shape and the cells remained viable after 24 hrs of culture. In addition, exposure of the three dimensional tissue to shipping conditions did not have a great impact on viability of cells.

Biochemical comparisons made between microsphere cultures and monolayer cultures over a 4-week culture period showed that the rate of cellular proliferation (as assessed as DNA) was equivalent; however, the overall cell mass was increased in the three dimensional tissues (see Table III). MTT reduction showed a strong correlation with DNA quantification. In all, the human SMC cultures produced nearly 10-fold more VEGF than the human DmFb cultures. The three dimensional tissues also appear to maintain their viability over time from 2 to 4 weeks.

**Table III*. Comparison of microsphere cultures with monolayer cultures.**

| **Monolayer** | **VEGF (ng/ugDNA)** | | **DNA(ug)** | | **MTT (abs 540)** | |
|---|---|---|---|---|---|---|
| | ***2wk*** | ***4wk*** | ***2wk*** | ***4wk*** | ***2wk*** | ***4wk*** |
| Human DmFb | 0.4+/-0.007 | 0.9+/-.05 | 1.7+/-0.7 | 1.9+/-1.2 | 0.7+/-0.04 | 0.63±/-0.4 |
| Canine SMC | 0.2+/-0.1 | 0.5+/-0.4 | 4.7+/-3.1 | 6.8+/-6.9 | ND | ND |
| Human SMC | 4.3+/-2.0 | ND | 0.8+/-0.5 | ND | 0.4+/-0.03 | 0.5+/-0.08 |
| | | | | | | |

| **Non-porous Microspheres** | **VEGF (ng/ugDNA)** | | **DNA(ug)** | | **MTT (abs 540)** | |
|---|---|---|---|---|---|---|
| | ***2wk*** | ***4wk*** | ***2wk*** | ***4wk*** | ***2wk*** | ***4wk*** |
| Human DmFb | 0.3+/-0.1 | 0.63+/-.03 | 1.9+/-0.7 | 3.7+/-1.5 | 1.0+1-0.06 | 1.1+/-0.04 |
| Canine SMC | 0.1+/-0.05 | 0.7+/-0.2 | 6.8+/-1.9 | 2.7+/-0.6 | ND | ND |
| Human SMC | 2.6+/-0.7 | 1.0+/-0.15 | 1.0+/-0.05 | 1.9+/-0.2 | 0.5+/-0.07 | 0.5+/-.04 |
| | | | | | | |

| **Porous Microspheres** | **VEGF (ng/ugDNA)** | | **DNA(ug)** | | **MTT (abs 540)** | |
|---|---|---|---|---|---|---|
| | ***2wk*** | ***4wk*** | ***2wk*** | ***4wk*** | ***2wk*** | ***4wk*** |
| Human DmFb | 0.3+/-0.1 | 0.2+/-0.01 | 2.5+/-1.5 | 2.8+/-1.3 | 0.9+/-0.16 | 1.1+/-0.13 |
| Canine SMC | 0.3+/-0.1 | 0.5+/-0.1 | 5.6+/-1.9 | 3.9+/-0.4 | ND | ND |
| Human SMC | 2.2+/-0.8 | 0.7+/-0.1 | 0.8+/-0.1 | 1.5+/-0.1 | 0.3+/-0.18 | 0.5+/-0.04 |

| | | | | | | |
|---|---|---|---|---|---|---|
| ND = not yet determined *Data were obtained from 24-well static cultures after seeding 1x10⁶ cells with 1mg of microspheres per well. | | | | | | |

A significant amount of VEGF factor was observed in the three dimensional tissues prepared on non-porous beads relative to the monolayer control cultures. Although a surprising lack of collagen deposition was observed in the three dimensional tissues, this was not deemed a negative outcome for the compositions because it has not yet been determined what role extracellular matrix (ECM) will play with respect to biomechanical characteristics or in vivo performance.

### 6.2 Example 2: Three Dimensional Tissues Formed With Matted Fibers

Studies were performed using small pieces of PGA felt (Albany International, Prodesco). When grown in culture, these pieces of felt contract into small spherical tissues that can be injected. DmFb cultured on PGA felt scaffolds appeared to have reduced VEGF levels compared to microsphere cultures; however, the amount of angiogenic factor secretion was still considered significant enough for analysis of vessel development in the CAM assay.

Studies with both DmFb and SMC showed extensive contraction of the original felt scaffolding, an ability for the tissues to pass through an 18-G needle, and VEGF secretion from the three dimensional tissues even after 11 weeks in culture. These results suggest its applicability as injectable compositions.

### 6.3 Example 3: Three Dimensional Tissues Formed With Threads/Sutures

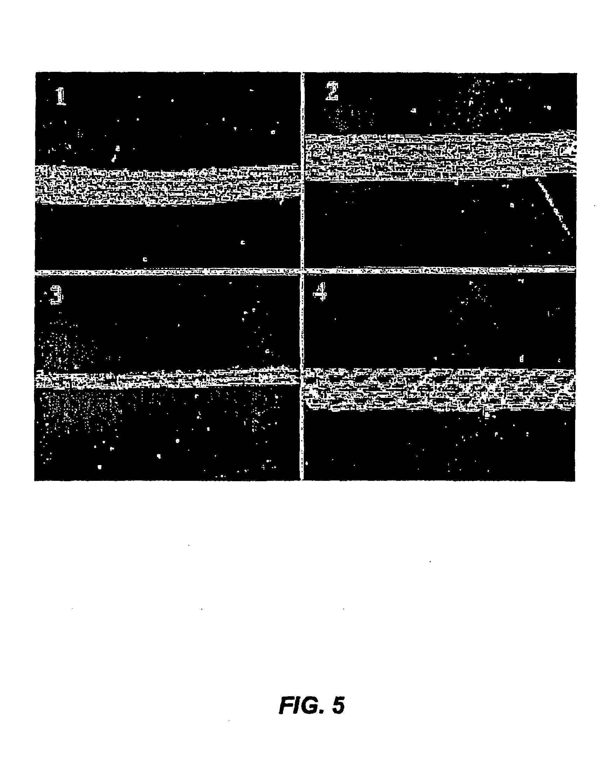

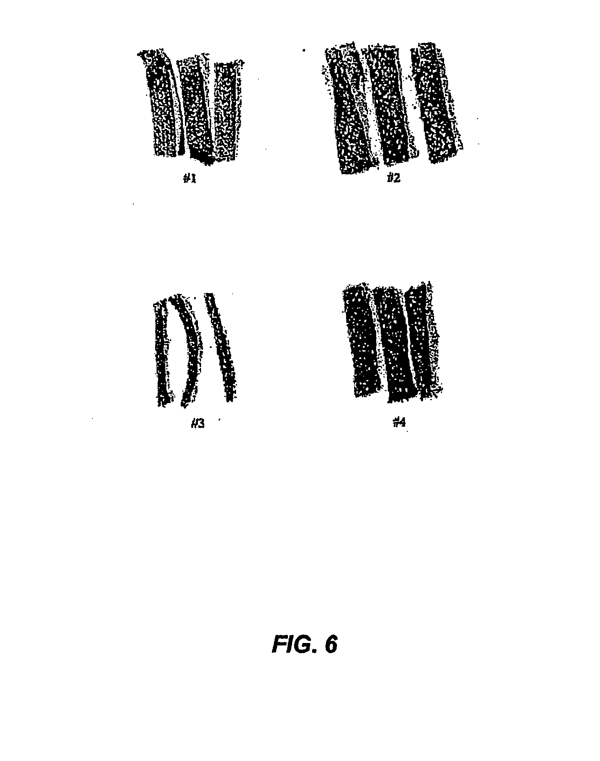

The braided scaffolds were made out of PLGA and essentially very small diameter, braided tubes. The four samples tested differed in the number of carriers (longitudinal fibers) and the number of axials (circumferential fibers). The samples were: (1) 24 carriers, 12 axials with a high braid angle (250 ppi); (2) 24 carriers, 12 axials with a low braid angle (200 ppi); (3) 8 carriers, 12 axials; and (4) 8 carriers, 24 axials. They all varied in diameter from 0.5 nun to 2.0 mm. A 24 hour seeding study was performed on these materials using canine SMC. Three seeding methods were used at a seeding density of 1 x 10⁵ cells per 1-inch length of material. The materials were cut into 1-inch pieces and seeded by various methods: (1) tumble seeding method (in a 1.5-ml conical tube), (2) laid in a trough with cells added to the trough, or (3) cells were injected with a needle into the lumen of the braid. MTT staining was performed after 24 hours and it appeared that only a few cells adhered to sample #4 (24 carriers, 12 axials); samples #1-3 had no observable MTT staining. This experiment was repeated using smaller volumes of media and a higher cell number (1 x 10⁶) to maximize attachment with improved results. Sample #4 continued to outperform the other designs. The remaining lengths of materials were sterilized for long-term culture studies using the best method of seeding (*i.e*., tumble seeding, which exhibited the greatest MTT staining). Samples were processed biochemically and histologically after 1 and 2 weeks of culture. Visual and biochemical results indicate that MTT staining was similar on all four sutures, slightly decreasing from 1 week to 2 weeks, while DNA increased from 1 to 2 weeks (Figures 5 and 6). After 2 weeks of growth, there was no significant collagen production as measured by hydroxyproline.

Growth of human DmFb on Prodesco® braided suture material was also examined. After three weeks in culture, nearly every PGA fiber was associated with cells (evident as dark-staining nuclei) and ECM formed (dark stained mass in center) on regions of the suture material. These results suggest that cells populate the entire walls of the tubular structures, begin filling intraluminal spaces, and are highly metabolic (via MTT reduction). The tensile strength of the cultured braided sutures, however, was considerably reduced after three weeks in culture. The reduction in tensile strength, however, can be addressed by modifications to the base polymer or to design improvements during manufacturing.

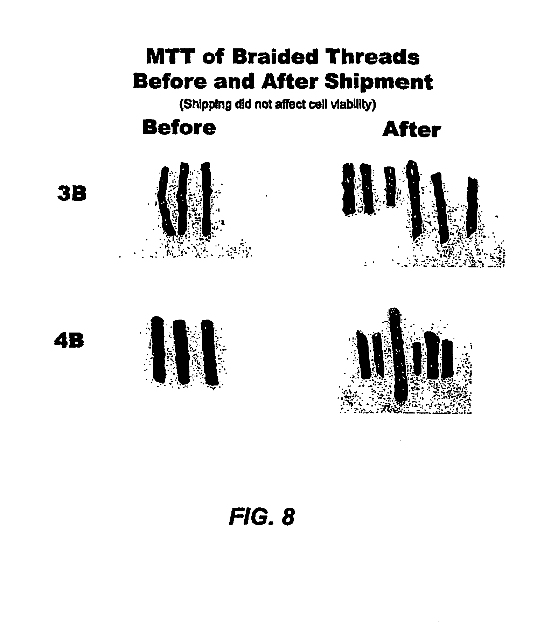

The three dimensional tissues of braided thread were subjected to shipment conditions, similar to that used to test the three dimensional tissues prepared using microspheres. Exposure of the three dimensional tissues of braided thread to shipping conditions did not dramatically influence cell viability (FIG. 8).

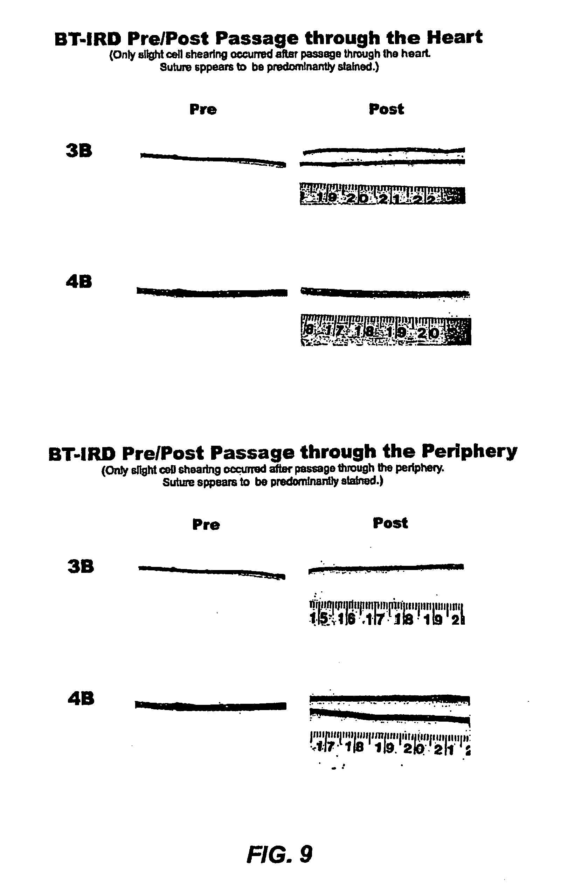

The effect of passing the three dimensional tissue of braided thread through muscle tissue is shown in FIG. 9. Cells remained on the braided thread and were viable after passage through both cardiac and peripheral muscle.

### 6.4 Example 5: Analysis of Growth Factors Produced by Minimally Invasive Tissue Constructs

Human dermal fibroblast and SMC cells cultured on Alkermes beads or felt under static conditions for 8 weeks were assessed for growth factor production. Specific messenger RNAs were estimated by quantitative RT-PCR using the ABI TaqMan method (Perkin-Elmer, Foster City, CA). RNA was extracted from the cells using a Rapid RNA Purification Kit (Amresco, Solon, OH, USA). The RNA was reverse transcribed using Superscript II (Life Technologies, Grand Island, NY) with random hexamer primers (Sigma, St. Louis, MO, USA). Amplification of samples of cDNA containing 200 ng total RNA was detected in real time and compared with the amplification of plasmid-derived standards for specific mRNA sequences using a copy number over a range of 5 orders of magnitude with 40-4,000,000/ reaction. In purification and the efficiency of reverse transcription, mRNA sequences for PDGF B chain, VEGF or TGFβ1 were added to RNA isolations, and their yield measured by the TaqMan procedure. The control mRNA sequences were obtained by T7 RNA polymerase transcription ofplasmids containing the corresponding sequence. The values were normalized using glyceraldehydes 3-phosphate dehydrogenase as a control.

For assessing the growth factors produced by the three dimensional tissues, the medium supernatants were analyzed by ELISA. Growth factor production was also determined for three lots of Dermagraft®. For the Dermagraft, the material was thawed according to manufacturer's instructions and laser-cut to 11mm x 11mm squares. Single squares were incubated with 1 ml of growth medium for 24 or 48 hours after thaw. Medium supernatants were analyzed by ELISA as indicated above. In addition, since basic Fibroblast Growth Factor (βFGF) is tightly bound to the extracellular matrix, single squares were extracted with 2M NaCl and dialyzed against PBS. The dialysate was then analyzed for the presence of βFGF.

In preliminary assays for growth factor production, there was significant variability in the factors secreted and the amounts secreted from cell type to cell type. Furthermore, there was variability in factors secreted by the same cell type on different scaffolds. These results indicate that growth factor analysis potentially can be used as release criteria, but must be tailored for specific cell type and scaffold.

### 6.5 Example 6: Injection of Three-Dimensional Stromal Tissues in an Ischemic Mouse Hind Limb Model

The mouse hindlimb ischemia model was developed in two different strains, the C57BL/6 and Balb/C. This model consists of ligating the artery and vein proximal to the bifurcation of the arteria profunda femoris and again at a site 5-7 mm distal. While both strains have been used in the literature, the Balb/C mouse has been found to collateralize less than other strains. Therefore, the Balb/C mouse strain was chosen for further studies.

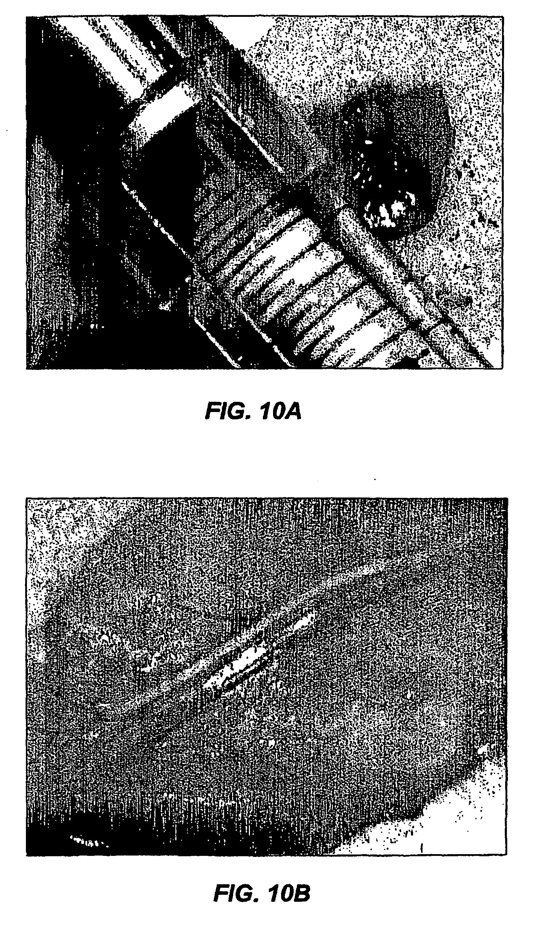

The mouse hindlimb ischemia model was used to evaluate the ability of minimally invasive constructs to induce angiogenesis in vivo when implanted into ischemic peripheral tissues. Ischemia was induced in two animals and the animals injected with a composition of smooth muscle cells cultured on Alkermes® beads. For a control, ischemia was induced in the animals but left untreated. Cells cultured on beads were successfully injected through a 24 gauge Hamilton syringe; however, approximately 50% of the bead volume remained in the syringe (FIGS. 10A and 10B). All 20 µl of media was injected with approximately 10 µl of the 20 µl of bead volume being delivered into the ischemic muscle. To insure full delivery, pharmaceutically suitable delivery agents such as PEG hydrogels may be used to increase viscosity of the vehicle and provide greater bead delivery.

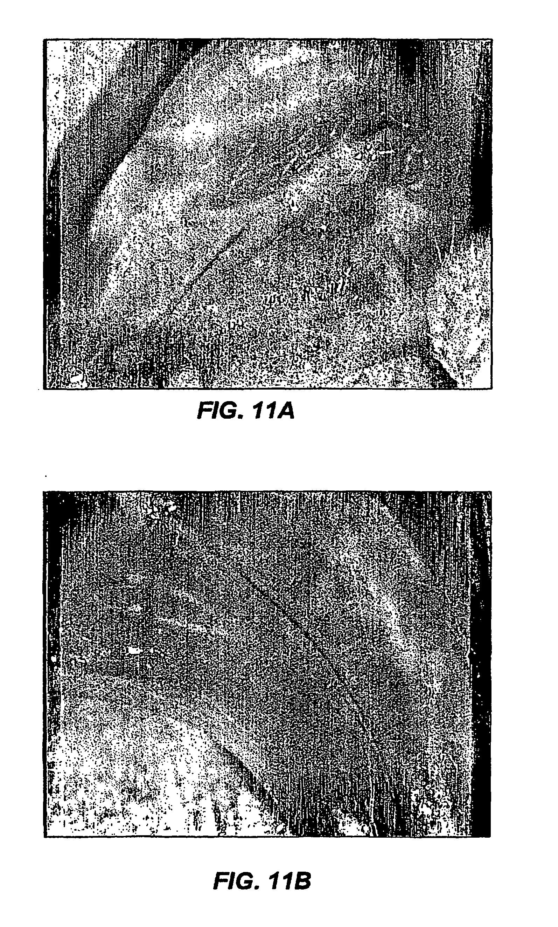

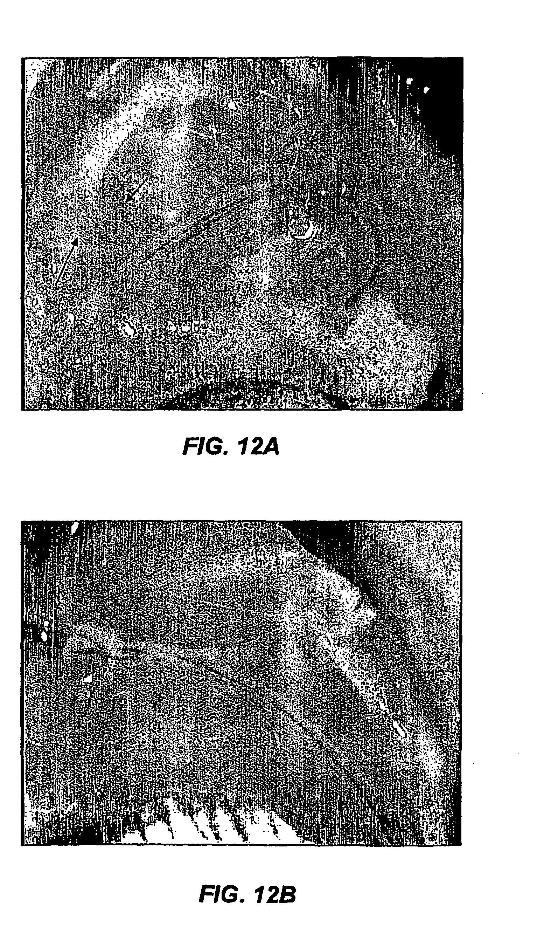

Observations 2 week after implantation demonstrated evidence of limited new microvessel formation (black arrows) in ischemic limbs treated with smooth muscle cells on Alkermes beads (FIGS. 12A and 12B) in comparison to control animals with ischemia-only limbs (FIGS. 11A and 11B).

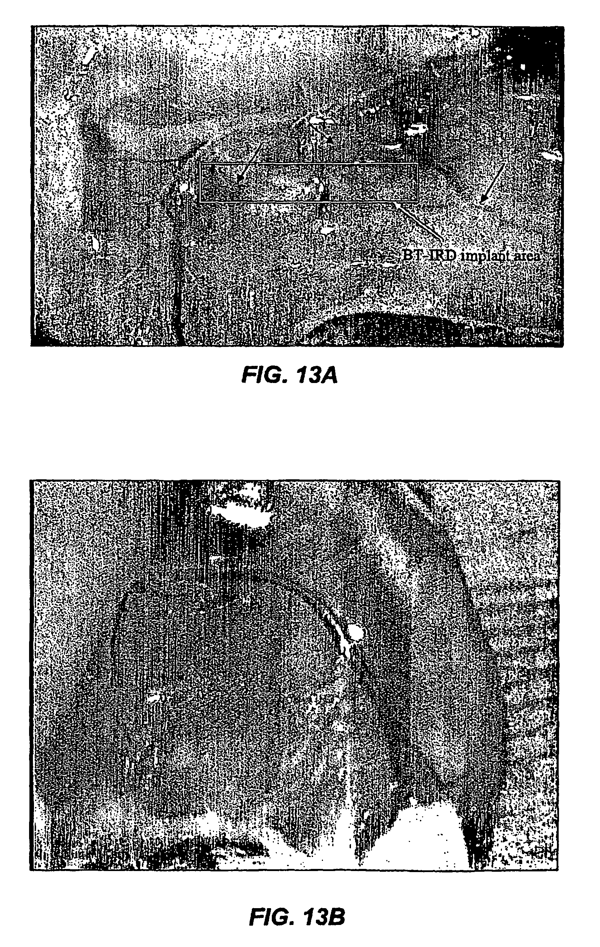

Studies using the mouse hindlimb ischemia model were also carried out with three dimensional tissues prepared using braided threads. Results suggest the presence of new microvessel formation surrounding the implants after 14 days of implantation (FIGS. 13A and 13B).
1. A composition, comprising:
   a cultured three dimensional tissue dimensioned for or so dimensioned as to permit penetration into tissues, wherein the three dimensional tissue comprises a scaffold of a biocompatible, nonliving material.
2. The composition of clause 1 in which the living cells comprise stromal cells.
3. The composition of clause 2 in which the stromal cells comprise fibroblasts.
4. The composition of clause 1 in which the livings cells comprise one or more of fibroblasts, smooth muscle cells, cardiac muscle cells, endothelial cells, pericytes, macrophages, monocytes, leukocytes, plasma cells, mast cells, or adipcytes.
5. The composition of clause 1 in which the living cells comprise mesenchymal stem cells.
6. The composition of clause 1 in which the scaffold comprises a population of microparticles.
7. The composition of clause 6 in which the microparticles are porous.
8. The composition of clause 6 in which the microparticles are nonporous.
9. The composition of clause 6 in which the microparticles comprise microspheres.
10. The composition of clause 6 in which the microparticles comprise a biodegradable material.
11. The composition of clause 10 in which the biodegradable material is polylactide, polyglycolic acid, polylactide-co-glycolic acid, trimethylene carbonate, and copolymers thereof in any combination and in any percent combination.
12. The composition of clause 10 in which the microparticle has a mean half life of about 14 days.
13. The composition of clause 10 in which the microparticle has a mean half life of about 28 days.
14. The composition of clause 10 in which the microparticle has a mean half life of about 42 days.
15. The composition of clause 10 in which the microparticle has a mean size of about 30 um.
16. The composition of clause 10 in which the microparticle has a mean size of about 60 um.
17. The composition of clause 10 in which the microparticle has a mean size of about 120 um.
18. The composition of clause 10 in which the microparticles comprise at least two different biodegradable materials.
19. The composition of clause 18 in which the different biodegradable materials form different layers on the microparticle.
20. The composition of clause 6 in which the microparticles are coated with an agent to enhance growth and attachment of cells.
21. The composition of clause 20 in which the agent is selected from collagen, elastin, glycoprotein, and glycosaminoglycans.
22. The composition of clause 1 in which the scaffold comprises a network of nonwoven filaments that form a particulate when cultured with the living cells.
23. The composition of clause 22 in which the network of nonwoven filaments comprises a felt.
24. The composition of clause 22 in which the nowoven filaments comprise a biodegradable filaments.
25. The composition of clause 23 in which the nonwoven filaments comprise at least two different biodegradable filaments.
26. The composition of clause 1 in which the scaffold comprises woven filaments that form a cord with interstitial spaces.
27. The composition of clause 26 in which the cord further comprises an internal luminal space formed by the woven filaments.
28. The composition of clause 26 in which the filaments are woven into a braid.
29. The composition of clause 28 in which the braid forms a sheath.
30. The composition of clause 26 in which the woven filaments comprise one or more biodegradable filaments.
31. The composition of clause 30, further comprising one or more non-biodegradable filaments.
32. The composition of clause 26 in which the scaffold is suitable for use as a surgical suture.
33. The composition of clause 1 in which the cell culture produces at least one WNT protein.
34. The composition of clause 33 in which the WNT protein is one or more of WNT5a., WNT7a, and WNT11.
35. The composition of clause 1 in which the cell culture produces VEGF.
36. A method of treating damaged tissue, comprising:
   administering the cell culture composition of any one of claims 1-35 in an amount effective to facilitate repair or regeneration of the damaged tissue,
37. The method of clause 36 in which the administration is by injection.
38. The method of clause 36 in which the administration is by a catheter.
39. The method of clause 36 in which the damaged tissue is an ischemic tissue.
40. The method of clause 39 in which the ischemic tissue is at least one of skeletal muscle, cardiac muscle, smooth muscle, or skin.
41. The method of clause 39 in which the ischemic tissue is the heart.
42. The method of clause 41 in which the ischemia tissue of the heart is the heart epicardium.
43. The method of clause 41 in which the ischemic tissue of the heart is the heart myocardium.
44. The method of clause 41 in which the ischemic tissue of the heart is the heart endocardium.
45. The method of clause 36 in which the damaged tissue is a chronically damaged tissue.
46. The method of clause 45 in which the chronically damaged tissue is the liver.
47. The method of clause 46 in which the damaged liver is fibrotic.
48. The method of clause 47 in which the damaged liver is cirrhotic.
49. The method of clause 47 in which the damaged liver is a liver with hepatitis.
50. The method of clause 36 in which the damaged tissue is the bone marrow.
51. The method of clause 50 in which the bone marrow has been treated with a cytotoxic agent prior to administration of the composition.
52. The method of clause 50 in which the bone marrow has been treated with radiation prior to administration of the composition.
53. A method of promoting vascularization in tissues, comprising; administering the cell culture composition of any one of claims 1-35 in an amount effective to induce formation of blood vessels.
54. The method of clause 53 in which the administration is by injection.
55. The method of clause 53 in which the administration is by a catheter.
56. The method of clause 53 in which the administration is into an ischemic tissue.
57. The method of clause 56 in which the ischemic tissue is at least one of skeletal muscle, cardiac muscle, smooth muscle, or skin.
58. The method of clause 56 in which the ischemic tissue is the heart.
59. The method of clause 58 in which the ischemic tissue of the heart is the heart epicardium.
60. The method of clause 58 in which the ischemic tissue of the heart is the heart myocardium.
61. The method of clause 53 in which the ischemic tissue of the heart is the heart endocardium.
62. A method for holding separated tissue together, comprising:
   joining the separated tissue with the surgical suture of claim 32.
63. The method of clause 62 in which the separated tissue is a wound.
64. The method of clause 63 in which the wound is a surgical incision.
65. The method of clause 64 in which the surgical incision is a resection.
66. The method of clause 64 in which the separated tissue is a blood vessel.
67. The method of clause 66 in which the blood vessel is a blood vessel graft.
68. The method of clause 67 in which the blood vessel graft is part of a coronary artery bypass graft.
69. The method of clause 62, further comprising attaching a three dimensional tissue to the site of the joined tissue.
70. A method of facilitating healing of anastomoses, comprising:
   applying the surgical suture of claim 32 in forming the anastomotic site.
71. The method of clause 70 in which the anastomosis is from a coronary artery bypass graft.
72. The method of clause 70 in which the anastomosis is from tissue resection.
73. The method of clause 70 in which the anastomosis is from an organ transplant.
74. The method of clause 73 in which the organ transplant is that of the heart, liver, kidney, or lung.
75. The method of clause 70, further comprising attaching a three dimensional tissue to the anastomotic site.

## Claims

1. A composition comprising microparticles of *in vitro*-cultured three dimensional tissue, wherein the microparticles are so dimensioned as to permit injection of the composition, and wherein the cultured tissue comprises:
a scaffold of a biocompatible, nonliving material;
mammalian stromal cells, wherein the stromal cells comprise fibroblasts, and
extracellular matrix elaborated by the cells in culture,
for use in the treatment of heart ischemia by injection into ischemic heart tissue.

2. Use of a composition comprising microparticles of *in vitro*-cultured three dimensional tissue, wherein the microparticles are so dimensioned as to permit injection of the composition, and wherein the cultured tissue comprises:
a scaffold of a biocompatible, nonliving material;
mammalian stromal cells, wherein the stromal cells comprise fibroblasts, and
extracellular matrix elaborated by the cells in culture,
in the manufacture of a medicament for treating heart ischemia by injection into ischemic heart tissue.

3. The composition for use according to claim 1 or the use according to claim 2, wherein the stromal cells comprise living fibroblasts.

4. The composition for use according to claim 1 or the use according to claim 2, wherein the microparticles are biodegradable.

5. The composition for use or use according to claim 4, wherein the microparticles are composed of a biodegradable material selected from polylactide, polyglycolic acid, polylactide-co-glycolic acid, trimethylene carbonate, and copolymers thereof in any combination and in any percent combination.

6. The composition for use according to claim 1 or the use according to claim 2, wherein the particles are nonbiodegradable.

7. The composition for use according to claim 1 or the use according to claim 2, wherein the scaffold comprises a network of nonwoven filaments that form microparticles when cultured with the stromal cells.

8. The composition for use according to claim 1 or the use according to claim 2, wherein the ischemic heart tissue is myocardium.

9. The composition for use according to claim 1 or the use according to claim 2, wherein the ischemic heart tissue is acutely ischemic.

10. The composition for use according to claim 1 or the use according to claim 2, wherein the ischemic heart tissue is chronically ischemic.

11. The composition for use or use according to any one of the preceding claims, wherein the microparticles of the composition have an average particle diameter between about 10 µm and about 1000 µm.

12. The composition for use or use according to any one of the preceding claims, wherein the particles are porous.

13. The composition for use or use according to any one of the preceding claims, wherein the particles are nonporous.

14. The composition for use or use according to any one of the preceding claims, wherein the fibroblasts are human fibroblasts.

15. The composition for use or use according to any one of the preceding claims, wherein the human fibroblasts are neonatal dermal fibroblasts.

## Patentansprüche

1. Zusammensetzung, die Mikroteilchen von in vitro kultiviertem dreidimensionalem Gewebe aufweist, wobei die Mikroteilchen so dimensioniert sind, dass sie die Injektion der Zusammensetzung zulassen, und wobei das kultivierte Gewebe aufweist:
ein Gerüst aus einem biokompatiblen, unbelebten Material;
Säugetier-Stromazellen, wobei die Stromazellen Fibroblasten aufweisen, und
eine durch die Zellen in Kultur produzierte extrazelluläre Matrix,
zur Verwendung bei der Behandlung von Herzischämie durch Injektion in ischämisches Herzgewebe .

2. Verwendung einer Zusammensetzung, die Mikroteilchen von in vitro kultiviertem dreidimensionalem Gewebe aufweist, wobei die Mikroteilchen so dimensioniert sind, dass sie die Injektion der Zusammensetzung zulassen, und wobei das kultivierte Gewebe aufweist:
ein Gerüst aus einem biokompatiblen, unbelebten Material;
Säugetier-Stromazellen, wobei die Stromazellen Fibroblasten aufweisen, und
eine durch die Zellen in Kultur produzierte extrazelluläre Matrix,
bei der Herstellung eines Medikaments zur Behandlung von Herzischämie durch Injektion in ischämisches Herzgewebe.

3. Zusammensetzung zur Verwendung nach Anspruch 1 oder Verwendung nach Anspruch 2, wobei die Stromazellen lebende Fibroblasten aufweisen.

4. Zusammensetzung zur Verwendung nach Anspruch 1 oder Verwendung nach Anspruch 2, wobei die Mikroteilchen biologisch abbaubar sind.

5. Zusammensetzung zur Verwendung oder Verwendung nach Anspruch 4, wobei die Mikroteilchen aus einem biologisch abbaubaren Material bestehen, das unter Polylactid, Polyglycolsäure, Polylactid-Co-Glycolsäure, Trimethylencarbonat und Copolymeren davon in beliebiger Kombination und in beliebigen Anteilen ausgewählt ist.

6. Zusammensetzung zur Verwendung nach Anspruch 1 oder Verwendung nach Anspruch 2, wobei die Teilchen nicht biologisch abbaubar sind.

7. Zusammensetzung zur Verwendung nach Anspruch 1 oder Verwendung nach Anspruch 2, wobei das Gerüst ein Netzwerk von Vliesfasern aufweist, die Mikroteilchen bilden, wenn sie mit den Stromazellen kultiviert werden.

8. Zusammensetzung zur Verwendung nach Anspruch 1 oder Verwendung nach Anspruch 2, wobei das ischämische Herzgewebe Myokardgewebe ist.

9. Zusammensetzung zur Verwendung nach Anspruch 1 oder Verwendung nach Anspruch 2, wobei das ischämische Gewebe akut ischämisch ist.

10. Zusammensetzung zur Verwendung nach Anspruch 1 oder Verwendung nach Anspruch 2, wobei das ischämische Herzgewebe chronisch ischämisch ist.

11. Zusammensetzung zur Verwendung oder Verwendung nach einem der vorstehenden Ansprüche, wobei die Mikroteilchen der Zusammensetzung einen mittleren Teilchendurchmesser zwischen etwa 10 µm und etwa 1000 µm haben.

12. Zusammensetzung zur Verwendung oder Verwendung nach einem der vorstehenden Ansprüche, wobei die Teilchen porös sind.

13. Zusammensetzung zur Verwendung oder Verwendung nach einem der vorstehenden Ansprüche, wobei die Teilchen nicht porös sind.

14. Zusammensetzung zur Verwendung oder Verwendung nach einem der vorstehenden Ansprüche, wobei die Fibroblasten humane Fibroblasten sind.

15. Zusammensetzung zur Verwendung oder Verwendung nach einem der vorstehenden Ansprüche, wobei die humanen Fibroblasten neonatale Hautfibroblasten sind.

## Revendications

1. Composition comprenant des microparticules de tissus tridimensionnels cultivés in vitro, les microparticules étant dimensionnées de sorte à permettre l'injection de la composition, les tissus cultivés comprenant :
un échafaudage de matériau non vivant biocompatible ;
des cellules stromales mammifères, les cellules stromales comprenant des fibroblastes ; et
une matrice extracellulaire élaborée par les cellules en culture ;
destinée à une utilisation dans le traitement d'une ischémie cardiaque par injection dans les tissus cardiaques ischémiques.

2. Utilisation d'une composition comprenant des microparticules de tissus tridimensionnels cultivés in vitro, les microparticules étant dimensionnées de sorte à permettre l'injection de la composition, les tissus de culture comprenant :
un échafaudage de matériau non vivant biocompatible ;
des cellules stromales mammifères, les cellules stromales comprenant des fibroblastes ; et
une matrice extracellulaire élaborée par les cellules en culture ;
dans la production d'un médicament pour traiter l'ischémie cardiaque par injection dans les tissus cardiaques ischémiques.

3. Composition pour utilisation selon la revendication 1, ou utilisation selon la revendication 2, dans laquelle les cellules stromales comprennent des fibroblastes vivants.

4. Composition pour utilisation selon la revendication 1 , ou utilisation selon la revendication 2, dans laquelle les microparticules sont biodégradables.

5. Composition pour utilisation ou utilisation selon la revendication 4, dans laquelle les microparticules sont composées d'un matériau biodégradable sélectionné parmi la polylactide, l'acide polyglycolique, l'acide de polylactide-co-glycolique, le carbonate de triméthylène et des copolymères de ces substances dans une quelconque combinaison et dans un quelconque pourcentage de combinaison.

6. Composition pour utilisation selon la revendication 1, ou utilisation selon la revendication 2, dans laquelle les particules ne sont pas biodégradables.

7. Composition pour utilisation selon la revendication 1, ou utilisation selon la revendication 2, dans laquelle l'échafaudage comprend un réseau de filaments non tissés formant des microparticules lors d'une culture avec des cellules stromales.

8. Composition pour utilisation selon la revendication 1, ou utilisation selon la revendication 2, dans laquelle les tissus cardiaques ischémiques sont des tissus myocardiques.

9. Composition pour utilisation selon la revendication 1, ou utilisation selon la revendication 2, dans laquelle les tissus cardiaques ischémiques présentent une ischémie aigue.

10. Composition pour utilisation selon la revendication 1, ou utilisation selon la revendication 2, dans laquelle les tissus cardiaques ischémiques présentent une ischémie chronique.

11. Composition pour utilisation ou utilisation selon l'une quelconque des revendications précédentes, dans laquelle les microparticules de la composition ont un diamètre de particule moyen compris entre environ 10 µm et environ 1000 µm.

12. Composition pour utilisation ou utilisation selon l'une quelconque des revendications précédentes, dans laquelle les particules sont poreuses.

13. Composition pour utilisation ou utilisation selon l'une quelconque des revendications précédentes, dans laquelle les particules sont non poreuses.

14. Composition pour utilisation ou utilisation selon l'une quelconque des revendications précédentes, dans laquelle les fibroblastes sont des fibroblastes humains.

15. Composition pour utilisation ou utilisation selon l'une quelconque des revendications précédentes, dans laquelle les fibroblastes humains sont des fibroblastes dermiques néonataux.
